# EUROPEAN PATENT APPLICATION

(11) **EP 3 260 446 A1**
(43) Date of publication of application: **27.12.2017**
(21) Application number: 16176168.9
(22) Date of filing: 24.06.2016
(51) Int. Cl.: C07D 239/22, C07D 239/10, C07D 405/04, C07D 409/04, C07C 275/12

(54) **ENANTIOSELECTIVE SYNTHESIS OF DIHYDROPYRIMIDINONES AND HEXAHYDROPYRIMIDINONES**

(71) Applicant: Universitat de les Illes Balears, 07122 Palma de Mallorca (ES)
(72) Inventor: Saá Rodríguez, José Manuel, 07122 Palma de Mallorca (ES); Mansilla Casatejada, Javier, 07180 Mallorca (ES); Lillo Dangla, Víctor Javier, 07006 Palma de Mallorca (ES)
(74) Representative: Manuel Illescas Asociados, S.L.

(57) **Abstract**

The present invention provides with a "one pot" easily scalable preparation process, or method to obtain enantiomerically enriched dihydropyrimidinones (DHPMs), in high yield and high enantiomeric purity, based on the concept of organocatalysis by a network of cooperative hydrogen bonds (NCHB). Said preparation process obtains enantiomerically enriched DHPMs without the use of metal-based catalysis and with the possibility of the recovery of the chiral organocatalyst comprising a NCHB used. The present invention also provides with new enantiomerically and diastereomerically enriched hexahydropyrimidinones (HHPMs), as well as a preparation process to obtain them also based on the concept of organocatalysis by a NCHB. Said preparation process obtains enantiomerically and diastereomerically enriched HHPMs without the use of metal-based catalysis and with the possibility of the recovery of the chiral organocatalyst comprising a NCHB used.

## Description

The present invention relates to the field of enantioselective synthesis of Biginelli's dihydropyrimidinones and hexahydropyrimidinones comprising the use of a chiral organocatalyst which provides a network of cooperative hydrogen bonds.

### BACKGROUND ART

Dihydropyrimidinones (DHPMs) are a family of compounds with a wide range of biological activity. Many of the DHPMs present pharmacological activity in very different health disorders such as arrhythmia, urinary incontinence, depression or sleep disorders, as well as acting as antihypertensive agents, calcium channel modulators, mitotic kinesin Eg5 inhibitors, melanin concentrating hormone receptor (MCHR1-R) antagonists or anticancer agents among others.

Some specific examples of DHPMs showing anticancer properties are disclosed as follows:

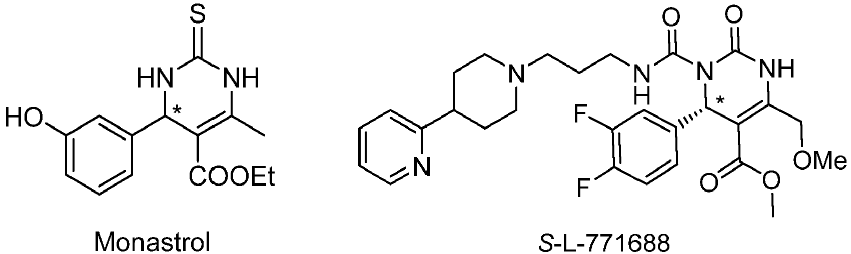

Other DHPMs are known to block calcium channels and are being explored for diseases as hypertension, arrhythmia, urinary incontinence, sleep disorders and depression, among other disorders.

Substituted 4-aryl-3,4-dihydropyrimidin-2(1H)-ones featured below herein are inhibitors of the human neutrophil elastase, therefore being active on chronic inflammatory processes, cardiovascular processes, myocardial infarct and acute coronary syndrome.

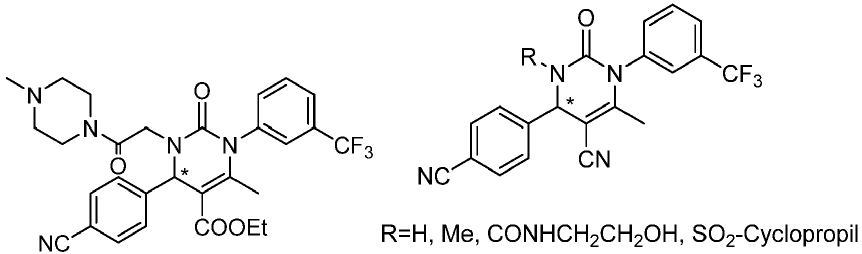

All these DHPMs are chiral substances which feature, at least, one stereogenic center at C₄ of the pyrimidinone moiety (marked with an asterisk * in all previously described compounds), wherein said stereogenic center, results in that the compound can exist as two stereoisomers called enantiomeric forms which are non-superimposable compounds or enantiomers (usually defined as R or S), or mixtures of them.

A mixture containing 50% of each of the enantiomers (R) and (S) is called a racemic mixture.

On the other hand, hexahydropyrimidinones (HHPMs) are closely related products to the DHPMs with the structural difference that HHPMs, being also chiral substances, feature not one but three stereogenic centers, in C₄, C₅ and C₆ (although IUPAC carbon numbering in said DHPMs and HHPMs is different due to the numbering priority rules). In fact, dehydration of HHPMs leads to the corresponding dihydropyrimidinones, DHPM. Some HHPMs analogues exhibit important cytotoxic properties and therefore, HHPMs and its analogues are a family of compounds which represent an important pharmacological lead for compounds with relevant pharmacological properties.

In the case of HHPMs wherein more than one stereogenic center is found, we define diastereomers as the stereoisomers having different configurations (R or S) at one or more (but not all) of the stereogenic centers. When two diastereomers differ from each other at only one stereogenic center are called epimers.

The most common method to obtain the racemic mixture of DHPMs is the three-component reaction, named after Pietro Biginelli, comprising the condensation in acid media of an aldehyde, urea and an acetoacetate. In this reaction, the addition of the urea and aldehyde results in an iminium intermediate which reacts with the acetoacetate, resulting in a product called Mannich adduct which features two stereogenic centers, and which eventually undergoes cyclization and dehydration to the corresponding DHPM. This reaction provides a racemic mixture of the Mannich adduct, which therefore also results in a racemic mixture of the final DHPM, and it is thus a non-enantioselective preparation process.

In many cases, the biological or pharmacological activity of one enantiomer is different from that of the other enantiomer and therefore, is important to provide methodologies or synthetic processes which are enantioselective and result in preferentially or exclusively only one of the two enantiomers.

Apart from the classic separation methods of enantiomers from a racemic mixture, the ideal method would be a direct, "one pot", scalable, enantioselective synthesis which leads to one specific enantiomer in high yield and high enantiomeric purity but which also features the added value of being atom economic, as according to the term coined by B.M. Trost (Science 1991,254, 1471).

Atom economy is a relevant factor to take into account when designing this ideal method. The efficiency of a process by is defined by its selectivity (regio, chemo and stereoselectivities) and its matter conservation; *i.e.,* the objective of atom economy is to maximize the number of atoms from the reactants appearing also in the desired final products. Atom economy is thus a different concept from chemical yield, because a high-yielding process can still result in substantial non-desired byproducts. An efficient process in terms of atom economy, not only provides a high yield of the desired final product but also provides a high selectivity and minimizes the existence of byproducts.

Enantiomeric purity is thus a relevant factor to take into account when designing said ideal method. It is usually specified with either one of the following measurements: a) the enantiomeric ratio or e.r (*i.e.,* the ratio o proportion of the two enantiomers present) or b) the enantiomeric excess or (e.e.) which can be defined as the ratio of the result of substracting the molar fraction of the major enantiomer from the molar fraction of the minor enantiomer and divide said result by the sum of the molar fractions of the major enantiomer and the molar fraction of the minor enantiomer multiplied by 100.

A number of direct, enantioselective synthesis of DHPMs have been published, employing chiral lanthanide complexes, chiral Lewis acids, chiral phosphoric acids as organocatalysts, bifunctional organocatalysts, or dual systems. Some of these methods make use of metals and are therefore less environmentally friendly, whereas other methods need of harsh or extreme conditions or feature a poor atom economy.

Stepwise approaches have also been reported that usually make use of an enantioselective Mannich reaction which is then followed by a number of additional steps. In fact, WO2007/011910 A2 provides with an enantioselective method to obtain DHPMs in which α-amidosulfones are used as an imine precursor to obtain the Mannich adduct and makes use of a cinchonine catalyst. After isolation of said Mannich adduct, it is then forced to undergo cyclization with the use of two extra steps comprising the use of an isocyanate in the presence of a Palladium catalyst, and the use of microwave energy. Therefore, this method to obtain DHPMs not only is not "one pot", and therefore less efficient and more difficult to scale-up industrially, but also features a poor atom economy and needs to make use of metal catalysts making it less environmentally friendly.

It is therefore widely recognized the unmet need for new efficient methodologies, both in terms of atom economy and energy, providing an efficient, environmentally friendly (non-metal catalysis) entry to enantiomerically pure DHPMs, which simplifies the number of synthetic operations to a "one-pot" methodology and allows for the recovery of the catalyst and its reutilization in subsequent batches without loss of efficiency.

To this end, the use of small organic molecules as catalysts (i.e. organocatalysts) capable of being recovered and subsequently reused in further batches is seen as a highly efficient and desirable catalytic plan which emulates those actually employed by enzymes.

Rawal et al. (c.f. Nature (2003) 424, 146 and Proc. Natl. Acad. Sci. (2004) 101, 5846) reported catalysis by pure hydrogen bond donors acting in a cooperative manner as according to Houk et al. (c.f. Org. Lett. (2008) 10, 2749), and Ooi et al. (c.f. Science, (2009) 326, 120) defined the importance of supramolecular recognition in trying to construct catalysts having a network of hydrogen bonds that cooperate in a stereocontrolling event, *i.e.* an enantioselective synthetic step.

J.M. Saá et al. (c.f. Angew. Chem. Int. Ed. (2016), 55, 4312) have shown that the hydrogen bond network present in compounds featuring an N,N'-bis(o-hydroxybenzyl)-1,2-diaminoethane scaffold, are capable to catalyze direct Mannich addition of acetoacetates and arylideneureas, producing Biginelli's DHPMs in high yield and enantiomeric excess.

Unfortunately, arylideneureas are extremely reactive species and impossible to store, and thus cannot be used in an easily scalable synthesis. Therefore, new methodologies are still needed for an efficient, environmentally friendly, "one-pot" synthetic process which allows for the recovery and reusing of the catalyst and leads to the enantioselective preparation process of the DHPMs in high yield and high enantiomeric purity.

### BRIEF DESCRIPTION OF THE INVENTION

The present disclosure provides with "one pot" easily scalable preparation processes, or methods to obtain enantiomerically enriched dihydropyrimidinones (DHPMs), in high yield and high enantiomeric excess, under mild operating conditions based on the concept of organocatalysis by a network of cooperative hydrogen bonds (NCHB). Said preparation processes obtain enantiomerically enriched DHPMs without the use of metal-based catalysis and with the possibility of the recovery and reutilization of said organocatalyst comprising a NCHB.

As described herein, the term "one pot" refers to a preparation process in which all steps can occur in the same reactor, there is no need to isolate intermediate compounds and all reactions may be carried out without the need of any intermediate separation step.

The present disclosure also provides with new enantiomerically and diastereomerically enriched hexahydropyrimidinones (HHPMs), as well as a "one pot" preparation process to obtain them, said preparation process also based on the concept of organocatalysis by a NCHB. Said preparation process obtains enantiomerically and diasteromerically enriched HHPMs in high yield and high enantiomeric and diastereomeric excess, without the use of metal-based catalysis and with the possibility of the recovery and reutilization of said organocatalyst comprising a NCHB.

A first aspect of the disclosure relates to a preparation process of a reaction crude comprising a compound of formula I: 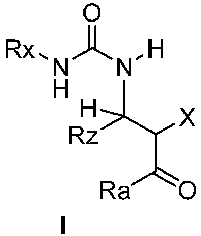 wherein said process is characterized by adding a compound of formula X-CH₂-C(O)Rₐ to a compound of formula II 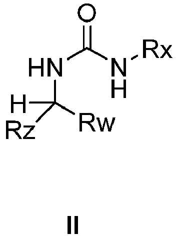 in the presence of a diastereomerically and enantiomerically enriched organocatalyst comprising a network of cooperative hydrogen bonds (NCHB) of formula III, a base and a solvent, wherein said preparation process is conducted maintaining a reaction temperature of between -80°C and 85°C;
(i) wherein,
   - Rₓ is selected independently from H, alkyl, aryl, heteroaryl or a fused aryl comprising 0-5 heteroatoms selected independently from nitrogen, oxygen or sulfur;
   - R_{z} is selected independently from H, heteroaryl, phenyl, substituted aryl, fused aryl comprising 0-5 heteroatoms selected independently from nitrogen, oxygen or sulfur; or a group -C(R")₃, wherein each group R" is independently selected from halogen, alkyl or aryl;
   - R_{w} is independently selected from halogen,-CN, -OCN, -SCN, -ONC, -SNC, -OR', -OC(O)R', -OC(O)OR', -OC(O)SR', -OC(S)OR', -OC(S)SR', -OC(O)N(R')₂, -OC(NR')N(R')₂, -OP(R')₂, -OP(O)R', -OP(O)OR', -OP(O)(OR')₂, -OP(S)R', -OP(S)OR', -OP(S)(OR')₂, -OP(S)SR', -OP(S)(SR')₂, -OP(O)N(R')₂, -OP(O)(NR')₂, -OP(S)N(R')₂, -OP(S)(NR')₂, -OP(O)₂OR', -OP(O)₂SR', -OP(O)₂N(R')₂, -OP(O)₂OP(O)₂OR', -OP(O)₂OP(O)₂SR', -OP(O)₂OP(O)₂N(R')₂, -OP(O)₂OP(O)₂OP(O)₂OR', -OP(O)₂OP(O)₂OP(O)₂SR', -OP(O)₂OP(O)₂OP(O)₂N(R')₂, -SR', -S(O)R', -S(O)₂R', -S(O)(NR')R', -S(O)₂OR', -S(O)₂N(R')₂, -S(NR')(NR')R', -S(S)₂R', -S(S)(NR')R' -S(S)₂R', -S(S)₂OR', -S(S)₂N(R')₂, -NHR', -N(R')₂, -NHC(O)R', -NR'C(O)R', -NR'C(O)OR', -NR'C(O)N(R')₂, -NR'C(S)R', -NR'C(S)OR', -NR'C(S)N(R')₂, -NHC(O)OR', -NHC(O)N(R')₂, -NHC(S)R', -NHC(S)OR', -NHC(S)N(R')₂, -N₃, -P(R')₂, -P(O)(R')₂, -P(OR')₂, -P(O)(OR')₂, -P(O)[N(R')₂]₂, -P(O)(OR')[N(R')₂], -P(S)(R')₂, -P(S)(OR')₂, -P(S)(SR')₂, or -P(S)[N(R')₂]₂; wherein each R' is independently selected from hydrogen, a metal, alkyl, halogen-alkyl, alkenyl, phenyl or substituted aryl;
   - X is selected independently from -C(O)OR, -C(O)SR, -C(S)OR, -C(S)SR, -C(O)NH₂, -C(O)NHR, -C(O)N(R)₂, -C(O)NHOR, -C(O)NROR, -C(S)NH₂, -C(S)NHR, -C(S)N(R)₂, -C(S)NHOR, -C(S)NROR, -P(O)(OR)₂, -P(O)OR, -P(O)(SR)₂, -P(O)SR, -P(S)(SR)₂, -P(S)SR, -P(O)(NH₂)₂, -P(O)(NHR)₂, -P(O)[N(R)₂]₂, -P(S)(NH₂)₂, -P(S)(NHR)₂, -CN, -NO₂, -SO₂R, -SOR or -P(S)[N(R)₂]₂, wherein each R is independently selected from alkyl, halogen-alkyl, alkenyl, a saturated or partially unsaturated cycle, a saturated or partially unsaturated heterocycle, an aryl, an heteroaryl or a fused aryl comprising 0-5 heteroatoms selected independently from nitrogen, oxygen or sulfur; and
   - Rₐ is selected independently from H, alkyl, halogen-alkyl, alkenyl, alkylalkoxy, alkylamine, alkylammonium, alkylphosphine, alkylphosphonium, a saturated or partially unsaturated cycle, a saturated or partially unsaturated heterocycle, an aryl, an heteroaryl or a fused aryl comprising 0-5 heteroatoms selected independently from nitrogen, oxygen or sulfur;
(ii) wherein in said diastereomerically and enantiomerically enriched organocatalyst comprising a NCHB of formula III: 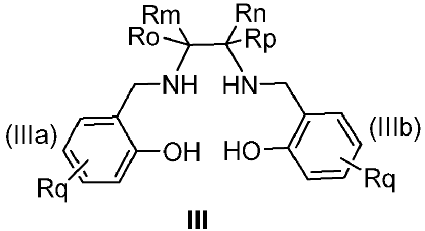
   - Rₘ, and Rₙ, or Rₒ and Rₚ, are linked forming a saturated or partially unsaturated C₃₋₁₂ ring; or are each independently selected from H, alkyl, aryl or heteroaryl; and
   - Rq is selected independently from H, hydroxyl, thiol, sulfone, amine, nitro, halogen, cyano, alkyl, alkylalkoxy, aryl or heteroaryl; or wherein each radical Rq of rings (IIIa) and (IIIb) is an aryl or an heteroaryl ring fused to each of said aryl rings (IIIa) and (IIIb) by two contiguous positions.

Accordingly, a second aspect of the disclosure relates to an enantioselective, easily scalable preparation process to obtain enantiomerically enriched dihydropyrimidinones (DHPMs) of formula IV: 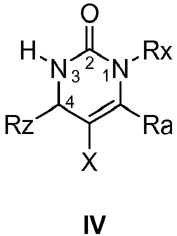 wherein said process comprises:
a) obtaining the reaction crude comprising the compound of formula I according to the preparation process described above herein; and
b) adding an acid to the reaction crude of step (a); and
wherein one DHPM enantiomer is obtained with enantiomeric excess greater than 0%.

Another further aspect of the invention relates to new enantiomerically and diastereomerically enriched hexahydropyrimidinones (HHPMs) of general formula V 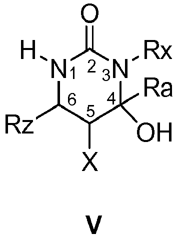 wherein
- Rₓ is selected independently from H, alkyl, aryl, heteroaryl or a fused aryl comprising 0-5 heteroatoms selected independently from nitrogen, oxygen or sulfur;
- R_{z} is selected independently from H, heteroaryl, phenyl, substituted aryl, fused aryl comprising 0-5 heteroatoms selected independently from nitrogen, oxygen or sulfur; or a group -C(R")₃, wherein each group R" is independently selected from halogen, alkyl or aryl;
- X is selected independently from -C(O)OR, -C(O)SR, -C(S)OR, -C(S)SR, -C(O)NH₂, -C(O)NHR, -C(O)N(R)₂, -C(O)NHOR, -C(O)NROR, -C(S)NH₂, -C(S)NHR, -C(S)N(R)₂, -C(S)NHOR, -C(S)NROR, -P(O)(OR)₂, -P(O)OR, -P(O)(SR)₂, -P(O)SR, -P(S)(SR)₂, -P(S)SR, -P(O)(NH₂)₂, -P(O)(NHR)₂, -P(O)[N(R)₂]₂, -P(S)(NH₂)₂, -P(S)(NHR)₂, -CN, -NO₂, -SO₂R, -SOR or -P(S)[N(R)₂]₂, wherein each R is independently selected from alkyl, halogen-alkyl, alkenyl, a saturated or partially unsaturated cycle, a saturated or partially unsaturated heterocycle, an aryl, an heteroaryl or a fused aryl comprising 0-5 heteroatoms selected independently from nitrogen, oxygen or sulfur; and
- Rₐ is selected independently from H, alkyl, halogen-alkyl, alkenyl, alkylalkoxy, alkylamine, alkylammonium, alkylphosphine, alkylphosphonium, a saturated or partially unsaturated cycle, a saturated or partially unsaturated heterocycle, an aryl, an heteroaryl or a fused aryl comprising 0-5 heteroatoms selected independently from nitrogen, oxygen or sulfur.

Another further aspect of the disclosure relates to a preparation process to produce enantiomerically and diastereomerically enriched hexahydropyrimidinones (HHPMs) of formula V wherein said process comprises:
a) obtaining the reaction crude comprising the compound of formula I according to the preparation process described above herein;
b) adding a base to the reaction crude of step (a); and
wherein one HHPM stereoisomer is obtained with a diastereomeric excess greater than 0% and an enantiomeric excess greater than 0%.

### DETAILED DESCRIPTION OF THE INVENTION

The enantioselective preparation processes of present disclosure, of both DHPMs of formula IV and HHPMs of formula V share a common synthetic concept wherein in a first step (a) a compound of formula II reacts with a compound of formula X-CH₂-C(O)Rₐ in the presence of a diastereomerically and enantiomerically enriched organocatalyst comprising a network of cooperative hydrogen bonds (NCHB) of formula III, a base and a solvent, and maintaining a temperature of between -80°C and 85°C.

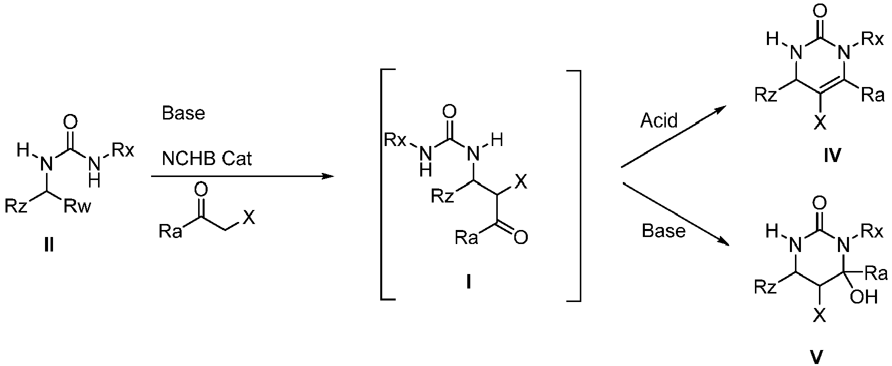

Said first reaction, shared in both processes where the DHPMs of formula IV and HHPMs of formula V of present disclosure are obtained, is a preparation process of a reaction crude comprising a compound of formula I, wherein said compound of formula I is the same common intermediate compound to obtain both the DHPMs of formula IV and HHPMs of formula V.

Acid treatment of said reaction crude results in enantiomerically enriched DHPMs of formula IV, and base treatment of the reaction crude results in the enantiomerically and diastereomerically enriched HHPMs of formula V.

As used herein a reaction crude is a mixture resulting of the preparation process wherein no steps of isolation or purification of the resulting products is conducted.

In some embodiments, the organocatalyst comprising a network of cooperative hydrogen bonds (NCHB) of formula III are enantiomerically and diastereomerically enriched, and the resulting DHPMs and HHPMs are enantiomerically enriched and enantiomerically and diastereomerically enriched, respectively.

The term enantiomerically enriched refers herein to a chiral substance presenting an enantiomeric excess greater than 0%.

Enantiomeric excess or (e.e.) is defined as the ratio obtained by dividing the result of subtracting the molar fraction of the major enantiomer from the molar fraction of the minor enantiomer by the sum of the molar fractions of the major enantiomer and the molar fraction of the minor enantiomer multiplied by 100.

In an analogous manner, as described herein, the term diastereomerically enriched refers to a chiral substance presenting a diasteromeric excess (d.e.) greater than 0%. Diastereomeric excess (d.e.) is defined as the ratio obtained by dividing the result of subtracting the molar fraction of the major diastereoisomer from the molar fraction of the minor diastereoisomer by the sum of the molar fractions of the major diastereoisomer and the molar fraction of the minor diastereoisomer multiplied by 100.

The DHPMs of formula IV feature two enantiomeric forms (R) and (S) corresponding to the two configurations of the stereogenic carbon C₄.

In some embodiments, the DHPM of formula IV obtained according to the present disclosure is enantiomerically enriched.

Said enantiomerically enriched DHPMs of formula IV are obtained with enantiomeric excess (ee) in reference to the stereogenic center present in C₄, wherein in some embodiments one DHPM enantiomer is obtained with an enantiomeric excess greater than 0%.

In some embodiments, the DHPM of formula IV obtained according to the present disclosure is enriched with the (R) C₄ enantiomer.

In some embodiments, the DHPM of formula IV obtained according to the present disclosure is enriched with the (S) C₄ enantiomer.

In some embodiments of the present disclosure, one DHPM enantiomer is obtained with an enantiomeric excess greater than 50%. In some embodiments of present disclosure, one DHPM enantiomer is obtained with an enantiomeric excess greater than 75%. In other embodiments of present disclosure, one DHPM enantiomer is obtained with an enantiomeric excess greater than 85%. In other embodiments of present disclosure, one DHPM enantiomer is obtained with an enantiomeric excess greater than 95%.

HHPMs of formula V feature eight stereoisomers corresponding to the two configurations of each of the three stereogenic carbons of positions C₄, C₅ and C₆.

In some embodiments, the HHPM of formula V obtained according to the present disclosure is enantiomerically and diastereomerically enriched.

Said enantiomerically and diastereomerically enriched HHPMs of formula V are obtained with enantiomeric excess (ee) and with diastereomeric excess (de) in reference to the stereogenic center present in C₄, and wherein in some embodiments one stereoisomer is obtained with a diastereomeric excess greater than 0% and an enantiomeric excess greater than 0%.

In some embodiments, the HHPM of formula V obtained according to the present disclosure is enriched with the *(4R, 5R,* 6*S*) stereoisomer.

In some embodiments, the HHPM of formula V obtained according to the present disclosure is enriched with the (4*S*, 5*S*, 6*R*) stereoisomer.

In some embodiments one stereoisomer of the enantiomerically and diastereomerically enriched HHPMs of formula V is obtained with enantiomeric excess greater than 0%. In some embodiments one stereoisomer is obtained with enantiomeric excess greater than 50%. In some embodiments one stereoisomer is obtained with enantiomeric excess greater than 75%. In some embodiments one stereoisomer is obtained with enantiomeric excess greater than 85%. In some embodiments one stereoisomer is obtained with enantiomeric excess greater than 95%.

In some embodiments of the present disclosure, one stereoisomer of the enantiomerically and diastereomerically enriched HHPMs of formula V is obtained with a diastereomeric excess greater than 50%. In some embodiments of present disclosure, one stereoisomer is obtained with a diastereomeric excess greater than 75%. In other embodiments of present disclosure, one stereoisomer is obtained with a diastereomeric excess greater than 85%. In other embodiments of present disclosure, one stereoisomer is obtained with a diastereomeric excess greater than 95%.

One embodiment of the preparation process of the reaction crude comprising the compound of formula I of present disclosure makes use of a diastereomerically and enantiomerically enriched organocatalyst comprising a network of cooperative hydrogen bonds (NCHB) of formula III, which comprises a N,N'-bis(o-hydroxybenzyl)-1, 2- diaminoethane moiety: 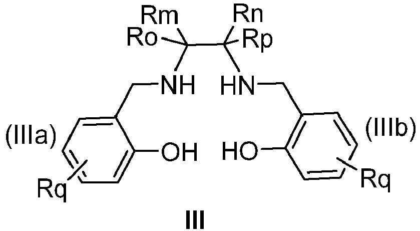 wherein
- Rₘ, and Rₙ, or Rₒ and Rₚ, are linked forming a saturated or partially unsaturated C₃-12 ring; or are each independently selected from H, alkyl, aryl or heteroaryl; and
- Rq is selected independently from H, hydroxyl, thiol, sulfone, amine, nitro, halogen, cyano, alkyl, alkylalkoxy, aryl or heteroaryl; or wherein each radical Rq of rings (IIIa) and (IIIb) is an aryl or an heteroaryl ring fused to each of said aryl rings (IIIa) and (IIIb) by two contiguous positions.

Said diastereomerically and enantiomerically enriched organocatalysts comprising a NCHB feature several functional groups comprising acidic hydrogen atoms located in such a way that are able to build a network of hydrogen bond donors and acceptors inducing cooperativity and adaptability to an incoming electrophile endowed with appropriate complementary acid/base groupings. Therefore, in the process of present disclosure the configuration featured by the chiral organocatalyst comprising a NCHB is efficiently transferred to the electrophile, *i.e.* the imine which is formed *in situ* from the compounds of formula II, and as a consequence, the nucleophile, *i.e.,* the enol form of the compounds of formula X-CH₂-C(O)Rₐ), can eventually discriminate the faces of said imine giving rise to an enantioselective process.

Said diastereomerically and enantiomerically enriched organocatalyst comprising a NCHB of formula III presents two stereogenic carbons linked to each of both amine groups.

One embodiment of the preparation process of the reaction crude comprising the compound of formula I of present disclosure makes use of said diastereomerically and enantiomerically enriched organocatalyst comprising a NCHB of formula III, wherein one stereoisomer of said diastereomerically and enantiomerically enriched organocatalyst comprising a NCHB of formula III is present in enantiomeric excess (ee).

In some embodiments, said diastereomerically and enantiomerically enriched organocatalyst comprising a NCHB of formula III is enriched with the (R,R) stereoisomer.

In some embodiments, said diastereomerically and enantiomerically enriched organocatalyst comprising a NCHB of formula III is enriched with the (S,S) stereoisomer.

The preparation process of the reaction crude comprising the compound of formula I of present disclosure makes use of said diastereomerically and enantiomerically enriched organocatalyst comprising a NCHB of formula III, wherein in some embodiments one stereoisomer is present in an enantiomeric purity greater than 75%. In some embodiments one stereoisomer is present in an enantiomeric purity greater than 85%. In some embodiments one stereoisomer is present in an enantiomeric purity greater than 90%. In other embodiments one stereoisomer is present in an enantiomeric purity greater than 95%. In other embodiments one stereoisomer is present in an enantiomeric purity greater than 99.5%.

The diastereomerically and enantiomerically enriched organocatalyst comprising a NCHB of formula III does not include metals and is recoverable and reusable in subsequent synthetic processes, as disclosed in Example 4, without significant performance loss in terms of yield or enantiomeric excess (ee) and diastereomeric excess (de).

Another embodiment of the preparation process of the reaction crude comprising the compound of formula I of present disclosure makes use of an enantiomerically enriched organocatalyst comprising a NCHB of formula III selected from:

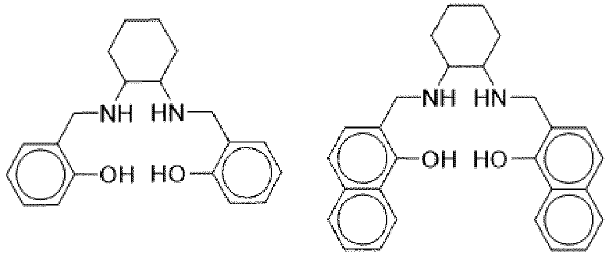

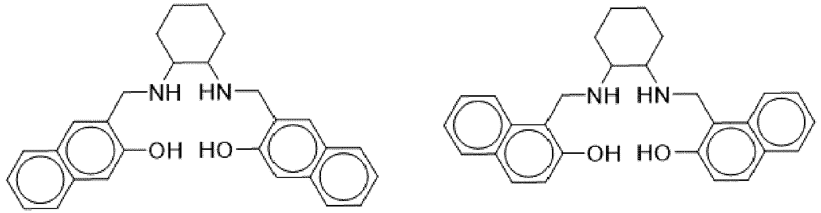

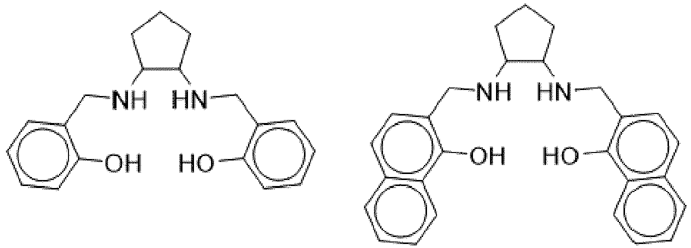

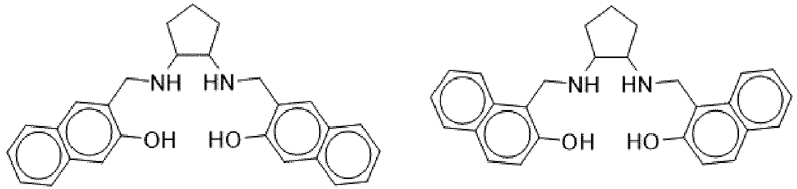

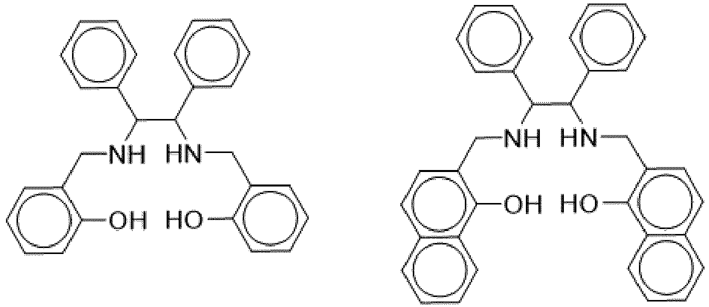

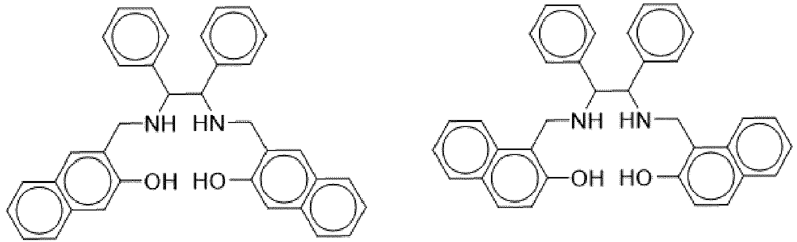

Another embodiment of the preparation process of the reaction crude comprising the compound of formula I of present disclosure makes use of a substochiometric amount of the diastereomerically and enantiomerically enriched organocatalyst comprising a NCHB of formula III as described in the embodiments of present disclosure above herein.

As used herein, substochiometric amounts refer to amounts of the diastereomerically and enantiomerically enriched organocatalyst comprising a NCHB of formula III which are lower than 1 mole equivalent relative to compound of formula II.

An embodiment of the preparation process of the reaction crude comprising the compound of formula I of present disclosure makes use of an amount of the diastereomerically and enantiomerically enriched organocatalyst comprising a NCHB of formula III which is lower than 0.5 mole equivalents. Another embodiment of present disclosure makes use of an amount of the diastereomerically and enantiomerically enriched organocatalyst comprising a NCHB of formula III which is lower than 0.1 mole equivalents. Another embodiment of present disclosure makes use of an amount of the diastereomerically and enantiomerically enriched organocatalyst comprising a NCHB of formula III which is lower than 0.05 mole equivalents.

Another embodiment of the preparation process of the reaction crude comprising the compound of formula I of present disclosure makes use of an amount of the diastereomerically and enantiomerically enriched organocatalyst comprising a NCHB of formula III as described in the embodiments of present disclosure above herein, equal or lower than 0.1 % molar.

Another embodiment of the preparation process of the reaction crude comprising the compound of formula I of present disclosure makes use of an amount of the diastereomerically and enantiomerically enriched organocatalyst comprising a NCHB of formula III as described in the embodiments of present disclosure above herein, equal or lower than 0.25 % molar.

Another embodiment of the preparation process of the reaction crude comprising the compound of formula I of present disclosure makes use of an amount of the diastereomerically and enantiomerically enriched organocatalyst comprising a NCHB of formula III as described in the embodiments of present disclosure above herein, equal or lower than 0.5 % molar.

Another embodiment of the preparation process of the reaction crude comprising the compound of formula I of present disclosure makes use of an amount of the diastereomerically and enantiomerically enriched organocatalyst comprising a NCHB of formula III as described in the embodiments of present disclosure above herein, equal or lower than 0.75 % molar.

Another embodiment of the preparation process of the reaction crude comprising the compound of formula I of present disclosure makes use of an amount of the diastereomerically and enantiomerically enriched organocatalyst comprising a NCHB of formula III as described in the embodiments of present disclosure above herein, equal or lower than 1 % molar.

One embodiment of the preparation process of the reaction crude comprising the compound of formula I of present disclosure makes use of a compound of formula II 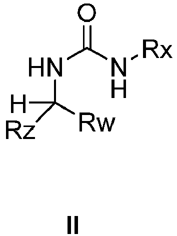 wherein,
- Rₓ is selected independently from H, alkyl, aryl, heteroaryl or a fused aryl comprising 0-5 heteroatoms selected independently from nitrogen, oxygen or sulfur;
- R_{z} is selected independently from H, heteroaryl, phenyl, substituted aryl, fused aryl comprising 0-5 heteroatoms selected independently from nitrogen, oxygen or sulfur; or a group -C(R")₃, wherein each group R" is independently selected from halogen, alkyl or aryl;
- R_{w} is independently selected from halogen,-CN, -OCN, -SCN, -ONC, -SNC, -OR', -OC(O)R', -OC(O)OR', -OC(O)SR', -OC(S)OR', -OC(S)SR', -OC(O)N(R')₂, -OC(NR')N(R')₂, -OP(R')₂, -OP(O)R', -OP(O)OR', -OP(O)(OR')₂, -OP(S)R', -OP(S)OR', -OP(S)(OR')₂, -OP(S)SR', -OP(S)(SR')₂, -OP(O)N(R')₂, -OP(O)(NR')₂, -OP(S)N(R')₂, -OP(S)(NR')₂, -OP(O)₂OR', -OP(O)₂SR', -OP(O)₂N(R')₂, -OP(O)₂OP(O)₂OR', -OP(O)₂OP(O)₂SR', -OP(O)₂OP(O)₂N(R')₂, -OP(O)₂OP(O)₂OP(O)₂OR', -OP(O)₂OP(O)₂OP(O)₂SR', -OP(O)₂OP(O)₂OP(O)₂N(R')₂, -SR', -S(O)R', -S(O)₂R', -S(O)(NR')R', -S(O)₂OR', -S(O)₂N(R')₂, -S(NR')(NR')R', -S(S)₂R', -S(S)(NR')R' -S(S)₂R', -S(S)₂OR', -S(S)₂N(R')₂, -NHR', -N(R')₂, -NHC(O)R', -NR'C(O)R', -NR'C(O)OR', -NR'C(O)N(R')₂, -NR'C(S)R', -NR'C(S)OR', -NR'C(S)N(R')₂, -NHC(O)OR', -NHC(O)N(R')₂, -NHC(S)R', -NHC(S)OR', -NHC(S)N(R')₂, -N₃, -P(R')₂, -P(O)(R')₂, -P(OR')₂, -P(O)(OR')₂, -P(O)[N(R')₂]₂, -P(O)(OR')[N(R')₂], -P(S)(R')₂, -P(S)(OR')₂, -P(S)(SR')₂, or -P(S)[N(R')₂]₂; wherein each R' is independently selected from hydrogen, a metal, alkyl, halogen-alkyl, alkenyl, phenyl or substituted aryl.

The term alkyl refers herein to a substituted or unsubstituted, linear or branched C₁₋₁₂ hydrocarbon chain which is completely saturated.

The term halogen-alkyl refers herein to a linear or branched C₁₋₁₂ hydrocarbon chain featuring at least one halogen substitution.

The term alkenyl refers herein to a substituted or unsubstituted, linear or branched C₁₋₁₂ hydrocarbon chain which features at least one unsaturation but which is not aromatic.

The term partially unsaturated cycle refers herein to a substituted or unsubstituted C₃₋₁₂ cycle group which includes at least one double or triple bond between ring atoms but which is not aromatic.

The term substituted aryl refers herein to an aromatic C₃₋₁₂ ring featuring at least one substitution.

The term saturated or partially unsaturated heterocycle refers herein to a substituted or unsubstituted 3-12 membered cycle group which may feature at least one unsaturation, but it is not aromatic, and features at least one heteroatom selected from N, O or S.

The term heteroaryl refers herein to a substituted or unsubstituted 3-12 membered aromatic cycle featuring at least one heteroatom selected from N, O or S.

The term fused aryl containing 0-5 heteroatoms refers herein to two or more substituted or unsubstituted aromatic rings linked to each other by two contiguous positions.

As described herein, possible substitutions include, but are not limited to hydroxyl, thiol, sulfone, amine, nitro, halogen, alkyl, alkenyl, alkoxy, alkylamine, alkylalcohol, alkylalkoxy or cyano.

One embodiment of the preparation process of the reaction crude comprising the compound of formula I of present disclosure makes use of a compound of formula II, wherein, Rₓ is H.

One embodiment of the preparation process of the reaction crude comprising the compound of formula I of present disclosure makes use of a compound of formula II, wherein, Rₓ is H or alkyl.

Another embodiment of the preparation process of the reaction crude comprising the compound of formula I of present disclosure makes use of a compound of formula II, wherein R_{z} is phenyl.

Another embodiment of the preparation process of the reaction crude comprising the compound of formula I of present disclosure makes use of a compound of formula II, wherein,
- when R_{z} is a substituted aryl, said aryl is substituted with one or more groups R_{b} 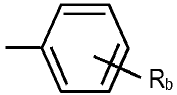 wherein R_{b} is independently selected from alkyl, alkoxy, halogen-alkyl, halogen, cyano, primary amine, secondary amines, tertiary amines or nitro; and
- when R_{z} is a fused aryl comprising 0-5 heteroatoms selected independently from nitrogen, oxygen or sulfur, said fused aryl is naphtyl.

Another embodiment of the preparation process of the reaction crude comprising the compound of formula I of present disclosure makes use of a compound of formula II, wherein R_{z} is phenylmethyl

Another embodiment of the preparation process of the reaction crude comprising the compound of formula I of present disclosure makes use of a compound of formula II, wherein R_{z} is fluorophenyl.

Another embodiment of the preparation process of the reaction crude comprising the compound of formula I of present disclosure makes use of a compound of formula II, wherein R_{z} is chlorophenyl.

Another embodiment of the preparation process of the reaction crude comprising the compound of formula I of present disclosure makes use of a compound of formula II, wherein R_{z} is bromophenyl.

Another embodiment of the preparation process of the reaction crude comprising the compound of formula I of present disclosure makes use of a compound of formula II, wherein R_{z} is cyanophenyl.

Another embodiment of the preparation process of the reaction crude comprising the compound of formula I of present disclosure makes use of a compound of formula II, wherein R_{z} is nitrophenyl.

Another embodiment of the preparation process of the reaction crude comprising the compound of formula I of present disclosure makes use of a compound of formula II, wherein R_{z} is trifluoromethylphenyl.

Another embodiment of the preparation process of the reaction crude comprising the compound of formula I of present disclosure makes use of a compound of formula II, wherein R_{z} is methoxyphenyl.

Another embodiment of the preparation process of the reaction crude comprising the compound of formula I of present disclosure makes use of a compound of formula II, wherein R_{z} is furanyl.

Another embodiment of the preparation process of the reaction crude comprising the compound of formula I of present disclosure makes use of a compound of formula II, wherein R_{z} is thienyl.

Another embodiment of the preparation process of the reaction crude comprising the compound of formula I of present disclosure makes use of a compound of formula II, wherein R_{z} is difluorophenyl.

Another embodiment of the preparation process of the reaction crude comprising the compound of formula I of present disclosure makes use of a compound of formula II, wherein R_{w} is selected from halogen, -OR', -OC(O)R', -S(O)₂R', -N₃, -OC(O)N(R')₂, -N(R')₂, -NHC(O)N(R')₂, -NR'C(O)N(R')₂ or -P(O)O(R')₂, and wherein each R' is independently selected from hydrogen, a metal, alkyl, halogen-alkyl, alkenyl, phenyl or substituted aryl.

Another embodiment of the preparation process of the reaction crude comprising the compound of formula I of present disclosure makes use of a compound of formula II, wherein R_{w} is halogen.

Another embodiment of the preparation process of the reaction crude comprising the compound of formula I of present disclosure makes use of a compound of formula II, wherein R_{w} is -S(O)₂R' or -OC(O)R' and wherein each R' is independently selected from hydrogen, a metal, alkyl, halogen-alkyl, alkenyl, phenyl or substituted aryl.

Another embodiment of the preparation process of the reaction crude comprising the compound of formula I of present disclosure makes use of a compound of formula II, wherein R_{w} is -S(O)₂R' and wherein each R' is independently selected from hydrogen, a metal, alkyl, halogen-alkyl, alkenyl, phenyl or substituted aryl.

Another embodiment of the preparation process of the reaction crude comprising the compound of formula I of present disclosure makes use of a compound of formula II, wherein R_{w} is -S(O)₂R' and R' is phenyl or substituted aryl.

Another embodiment of the preparation process of the reaction crude comprising the compound of formula I of present disclosure makes use of a compound of formula II, wherein R_{w} is -S(O)₂R' and R' is tolyl.

Several examples of compounds of formula II comprising a R_{w} group tosyloxy were synthetized in Example 1 following the method of Engberts et al. (Recl. Trav. Chim. Pays-Bas (1965) 84, 942-950) as disclosed in J.M. Saá et al. (c.f. Angew. Chem. Int. Ed. (2016), 55,4312).

One embodiment of the preparation process of the reaction crude comprising the compound of formula I of present disclosure makes use of compounds of formula X-CH₂-C(O)Rₐ wherein
- X is selected independently from -C(O)OR, -C(O)SR, -C(S)OR, -C(S)SR, -C(O)NH₂, -C(O)NHR, -C(O)N(R)₂, -C(O)NHOR, -C(O)NROR, -C(S)NH₂, -C(S)NHR, -C(S)N(R)₂, -C(S)NHOR, -C(S)NROR, -P(O)(OR)₂, -P(O)OR, -P(O)(SR)₂, -P(O)SR, -P(S)(SR)₂, -P(S)SR, -P(O)(NH₂)₂, -P(O)(NHR)₂, -P(O)[N(R)₂]₂, -P(S)(NH₂)₂, -P(S)(NHR)₂, -CN, -NO₂, -SO₂R, -SOR or -P(S)[N(R)₂]₂, wherein each R is independently selected from alkyl, halogen-alkyl, alkenyl, a saturated or partially unsaturated cycle, a saturated or partially unsaturated heterocycle, an aryl, an heteroaryl or a fused aryl comprising 0-5 heteroatoms selected independently from nitrogen, oxygen or sulfur; and
- Rₐ is selected independently from H, alkyl, halogen-alkyl, alkenyl, alkylalkoxy, alkylamine, alkylammonium, alkylphosphine, alkylphosphonium, a saturated or partially unsaturated cycle, a saturated or partially unsaturated heterocycle, an aryl, an heteroaryl or a fused aryl comprising 0-5 heteroatoms selected independently from nitrogen, oxygen or sulfur.

Another embodiment of the preparation process of the reaction crude comprising the compound of formula I of present disclosure makes use of compounds of formula X-CH₂-C(O)Rₐ wherein X is selected from X is selected independently from -C(O)OR, -C(O)SR, -C(S)OR, -C(S)SR, -C(O)NH₂, -C(O)NHR, -C(O)N(R)₂, -C(O)NHOR, -C(O)NROR, -C(S)NH₂, -C(S)NHR, -C(S)N(R)₂, -C(S)NHOR, -C(S)NROR, -P(O)(OR)₂, -P(O)OR, -P(O)(SR)₂, -P(O)SR, -P(S)(SR)₂, -P(S)SR, -P(O)(NH₂)₂, -P(O)(NHR)₂, -P(O)[N(R)₂]₂, -P(S)(NH₂)₂, -P(S)(NHR)₂, -CN, -NO₂, -SO₂R, -SOR or -P(S)[N(R)₂]₂, and wherein R is alkyl.

One embodiment of the preparation process of the reaction crude comprising the compound of formula I of present disclosure makes use of compounds of formula X-CH₂-C(O)Rₐ wherein X is -C(O)OR, and wherein each R is selected from alkyl, halogen-alkyl, alkenyl, a saturated or partially unsaturated cycle, a saturated or partially unsaturated heterocycle, an aryl, an heteroaryl or a fused aryl comprising 0-5 heteroatoms selected independently from nitrogen, oxygen or sulfur.

Another embodiment of the preparation process of the reaction crude comprising the compound of formula I of present disclosure makes use of compounds of formula X-CH₂-C(O)Rₐ wherein X is -C(O)OR and R is selected from a methyl or an ethyl group.

One embodiment of the preparation process of the reaction crude comprising the compound of formula I of present disclosure makes use of compounds of formula X-CH₂-C(O)Rₐ wherein Rₐ is alkyl.

One embodiment of the preparation process of the reaction crude comprising the compound of formula I of present disclosure makes use of compounds of formula X-CH₂-C(O)Rₐ wherein Rₐ is methyl or ethyl.

One embodiment of present disclosure is a preparation process of a reaction crude comprising a compound of formula I, wherein said process is characterized by adding a compound of formula X-CH₂-C(O)Rₐ, according to the embodiments described above herein, to the compounds of formula II, according to the embodiments described above herein, in the presence of an enantiomerically enriched chiral organocatalyst comprising a network of cooperative hydrogen bonds (NCHB) of formula III, a base and a solvent, wherein said preparation process is conducted maintaining a reaction temperature of between -80°C and 85°C.

One embodiment of present disclosure is a preparation process of a reaction crude comprising a compound of formula I, wherein said process comprises a solvent which includes, but is not limited to one or a mixture of polar aprotic solvents and/or halogenated hydrocarbon solvents. Examples of polar aprotic solvents include, but are not limited to, DMF, DMSO, THF, glyme, diglyme, MTBE, acetonitrile and acetone. Examples of halogenated hydrocarbon solvents include, but are not limited to, CH₂Cl₂, CHCl₃ and CCl₄.

One embodiment of the preparation process of the reaction crude comprising the compound of formula I of present disclosure make use of inorganic bases which include but are not limited to ammonium carbonate, sodium carbonate, potassium carbonate, cesium carbonate, lithium carbonate, ammonium phosphate, sodium phosphate, potassium phosphate, cesium phosphate or lithium phosphate.

One embodiment of the preparation process of the reaction crude comprising the compound of formula I of present disclosure make use of organic bases which include but are not limited to sodium, potassium, cesium or lithium alkoxydes, a primary amine, a secondary amine, a tertiary amine, amidines, 1,8-bis(dimethylamino)naphthalene, guanidines or aromatic amines.

Another embodiment of the preparation process of the reaction crude comprising the compound of formula I of present disclosure makes use of a base, wherein said base is an amidine selected from 1,8-diazabicyclo[5.4.0]undec-7-ene (DBU) or 1,5-diazabicyclo[4.3.0]nonene (DBN).

Another embodiment of the preparation process of the reaction crude comprising the compound of formula I of present disclosure makes use of a base, wherein said base is a guanidine selected from tetramethylguanidine (TMG) or tert-butyl-tetramethylguanidine (BTMG).

Another embodiment of the preparation process of the reaction crude comprising the compound of formula I of present disclosure makes use of a base, wherein said organic base is tetramethylguanidine (TMG).

Another embodiment of the preparation process of the reaction crude comprising the compound of formula I of present disclosure makes use of a base, wherein said organic base is a tertiary amine.

Another embodiment of the preparation process of the reaction crude comprising the compound of formula I of present disclosure makes use of a base, wherein said organic base is N,N'-diisopropylethylamine.

One embodiment of present invention is an enantioselective preparation process of the enantiomerically enriched DHPMs of formula IV, 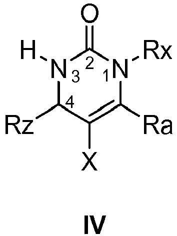 comprises:
- a step (a) which is the preparation process of a reaction crude comprising a compound of formula I, as described in the embodiments above herein, 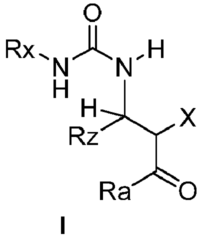 wherein said step (a) is characterized by adding a compound of formula X-CH₂-C(O)Rₐ, according to the embodiments described above herein, to a compound of formula II, according to the embodiments described above herein, and wherein said step (a) is conducted in the presence of the above described enantiomerically enriched organocatalyst comprising a NCHB of formula III, the above described bases and a solvent, maintaining a temperature of between -80°C and 85°C; and
- a step (b) wherein said step (b) is conducted by adding an acid to the reaction crude of step (a); and
wherein one DHPM enantiomer is obtained with enantiomeric excess greater than 0%.

One embodiment of present disclosure is an enantioselective preparation process of the enantiomerically enriched DHPMs of formula IV wherein said process is a "one pot" preparation process.

As described herein, one embodiment of the preparation process of the enantiomerically enriched DHPMs of formula IV makes use of acids which, as described herein, include but are not limited to hydroiodic acid, hydrobromic acid, hydrochloric acid, nitric acid, sulfuric acid, fluorosulfuric acid, perchloric acid, chloric acid, perbromic acid, bromic acid, alkyl sulfonic acids, trihalomethansulfonic acids, arylsulfonic acids, aryl substituted sulfonic acids, heteroarylsulfonic acids, fluroantimonic acid, formic acid, acetic acid, benzoic acids, propionic acid, alfa-halo acetic acids, alfa-halo aliphatic acids, alfa-hydroxy acids, beta-hydroxi acids, alfa-amino acids, beta-amino acids, oxalic acid, malonic acid, succinic acid, hydrofluoric acid, hydrocyanic acid, phosphoric acid, sulphurous acid, nitrous acid, iodic acid, carbonic acid, phenols, hydrogen sulfide, hydrogen sulfate anion, alkyl sulfenic acids, aryl sulfenic acids, aryl substituted sulfenic acids, alkyl sulfinic acids, aryl sulfinic acids, aryl substituted sulfinic acids,alkyl phosphonic acids, aryl phosphonic acids, aryl substituted phosphonic acids, alkyl phosphinic acids, aryl phosphinic acids, arylsubstituted phosphinic acids, ammonium salts or water.

Another embodiment of present disclosure is an enantioselective preparation process of the enantiomerically enriched DHPMs of formula IV, comprising a step (a) and a step (b) according to the embodiments described above herein, and wherein said step (a) is conducted maintaining a reaction temperature of between -10°C and 10°C.

Another embodiment of present disclosure is an enantioselective preparation process of the enantiomerically enriched DHPMs of formula IV, comprising a step (a) and a step (b) according to the embodiments described above herein, and wherein said step (a) is conducted maintaining a reaction temperature of between -10°C and 10°C for at least 8 hours.

Another embodiment of present disclosure is an enantioselective preparation process of the enantiomerically enriched DHPMs of formula IV, comprising a step (a) and a step (b) according to the embodiments described above herein, and wherein said step (b) is conducted by adding a hydrochloric acid solution to the reaction crude of step (a).

Another embodiment of present disclosure is an enantioselective preparation process of the enantiomerically enriched DHPMs of formula IV, comprising a step (a) and a step (b) according to the embodiments described above herein, wherein said step (a) is conducted maintaining a reaction temperature of between -10°C and 10°C for at least 8 hours and wherein said step (b) is conducted by adding a hydrochloric acid solution to the reaction crude of step (a).

Examples of "one pot" enantioselective preparation processes of the enantiomerically enriched DHPMs of formula IV according to the embodiments described above herein are included in Examples 2 and 3 of present disclosure. In Example 1 a range of compounds of formula II are synthesized.

In Example 2 a range of different enantiomerically enriched DHPMs of formula IV are synthesized according to the present disclosure.

Several lead enantiomerically enriched DHPMs were also synthetized in medium scale quantities (with a factor of x10) in Example 3 without significant impact to the yield or enantiomeric excess (ee) of the resulting DHPMs of formula IV.

In Example 4 the enantiomerically enriched chiral organocatalyst comprising a NCHB used in Example 3 was successfully recovered and reused to synthesize yet another DHPM of formula IV, without significant impact on yield or enantiomeric excess of the enantiomerically enriched DHPM of formula IV produced.

One embodiment of present disclosure is a preparation process of the enantiomerically and diastereomerically enriched HHPMs of formula V, 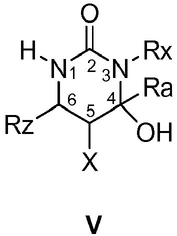 comprising:
- a step (a) which is the preparation process of a reaction crude comprising a compound of formula I as described in the embodiments above herein, 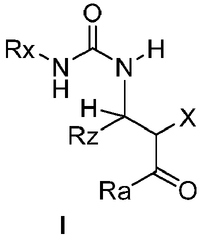 wherein said step (a) is characterized by adding a compound of formula X-CH₂-C(O)Rₐ, according to the embodiments described above herein, to a compound of formula II, according to the embodiments described above herein, and wherein said step (a) is conducted in the presence of the above described enantiomerically enriched organocatalyst comprising a NCHB of formula III, the above described bases and a solvent, maintaining a temperature of between -80°C and 85°C;
- a step (b) wherein said step (b) is conducted by adding a base to the reaction crude of step (a); and
wherein one HHPM stereoisomer is obtained with a diastereomeric excess greater than 0% and an enantiomeric excess greater than 0%.

Another embodiment of present invention is a preparation process of the enantiomerically and diastereomerically enriched HHPM of formula V, comprising a step (a) and a step (b) according to the embodiments described above herein, and wherein said step (a) is conducted maintaining a reaction temperature of between -10°C and 10°C.

Another embodiment of present disclosure is a preparation process of the enantiomerically and diastereomerically enriched HHPM of formula V, comprising a step (a) and a step (b) according to the embodiments described above herein, and wherein said step (a) is conducted maintaining a reaction temperature of between -10°C and 10°C for at least 8 hours.

Another embodiment of present disclosure is a preparation process of the enantiomerically and diastereomerically enriched HHPM of formula V, comprising a step (a) and a step (b) according to the embodiments described above herein, and wherein said step (b) is conducted by adding an inorganic base to the reaction crude of step (a).

Embodiments of the preparation process of the enantiomerically and diastereomerically enriched HHPMs of formula V of present disclosure make use in said step (b) of solid inorganic bases which include, but are not limited to ammonium carbonate, sodium carbonate, potassium carbonate, cesium carbonate, lithium carbonate, ammonium phosphate, sodium phosphate, potassium phosphate, cesium phosphate or lithium phosphate.

Another embodiment of present disclosure is a preparation process of the enantiomerically and diastereomerically enriched HHPM of formula V, comprising a step (a) and a step (b) according to the embodiments described above herein, and wherein said step (b) is conducted by adding an organic base to the reaction crude of step (a).

Embodiments of the preparation process of the enantiomerically and diastereomerically enriched HHPMs of formula V of present disclosure make use in said step (b) of organic bases which include, but are not limited to sodium, potassium, cesium or lithium alkoxydes, a primary amine, a secondary amine, a tertiary amine, amidines, 1,8-bis(dimethylamino)naphthalene, guanidines or aromatic amines.

Embodiments of the preparation process of the enantiomerically and diastereomerically enriched HHPMs of formula V of present disclosure make use in said step (b) of a base, wherein said base is an amidine selected from 1,8-diazabicyclo[5.4.0]undec-7-ene (DBU) or 1,5-diazabicyclo[4.3.0]nonene (DBN).

Embodiments of the preparation process of the enantiomerically and diastereomerically enriched HHPMs of formula V of present disclosure make use in said step (b) of a base, wherein said base is a guanidine selected from tetramethylguanidine (TMG) or tert-butyl-tetramethylguanidine (BTMG).

Another embodiment of present disclosure is a preparation process of the enantiomerically and diastereomerically enriched HHPM of formula V, comprising a step (a) and a step (b) according to the embodiments described above herein, wherein said step (a) is conducted maintaining a reaction temperature of between -10°C and 10°C for at least 8 hours and wherein said step (b) is conducted by adding tetramethylguanidine to the reaction crude of step (a).

Several enantiomerically and diastereomerically enriched HHPM of formula V were synthesized in enantiomeric and diasteromeric excess according to the embodiments described above herein in Example 5 of present invention.

One lead enantiomerically and diastereomerically enriched HHPMs of formula V was also synthetized, according to the embodiments described above herein, in medium scale quantity (with a factor of x10) in Example 6, without significant impact to the yield or enantiomeric excess (ee) or diastereomeric excess (de) of the resulting HHPMs of formula V.

Another embodiment of the present disclosure refers to enantiomerically and diastereomerically enriched HHPMs of general formula V 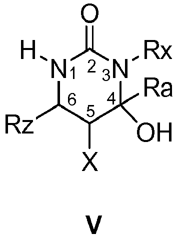 wherein,
- Rₓ is selected independently from H, alkyl, aryl, heteroaryl or a fused aryl comprising 0-5 heteroatoms selected independently from nitrogen, oxygen or sulfur;
- R_{z} is selected independently from H, heteroaryl, phenyl, substituted aryl, fused aryl comprising 0-5 heteroatoms selected independently from nitrogen, oxygen or sulfur; or a group -C(R")₃, wherein each group R" is independently selected from halogen, alkyl or aryl;
- X is selected independently from -C(O)OR, -C(O)SR, -C(S)OR, -C(S)SR, -C(O)NH₂, -C(O)NHR, -C(O)N(R)₂, -C(O)NHOR, -C(O)NROR, -C(S)NH₂, -C(S)NHR, -C(S)N(R)₂, -C(S)NHOR, -C(S)NROR, -P(O)(OR)₂, -P(O)OR, -P(O)(SR)₂, -P(O)SR, -P(S)(SR)₂, -P(S)SR, -P(O)(NH₂)₂, -P(O)(NHR)₂, -P(O)[N(R)₂]₂, -P(S)(NH₂)₂, -P(S)(NHR)₂, -CN, -NO₂, -SO₂R, -SOR or -P(S)[N(R)₂]₂, wherein each R is independently selected from alkyl, halogen-alkyl, alkenyl, a saturated or partially unsaturated cycle, a saturated or partially unsaturated heterocycle, an aryl, an heteroaryl or a fused aryl comprising 0-5 heteroatoms selected independently from nitrogen, oxygen or sulfur; and
- Rₐ is selected independently from H, alkyl, halogen-alkyl, alkenyl, alkylalkoxy, alkylamine, alkylammonium, alkylphosphine, alkylphosphonium, a saturated or partially unsaturated cycle, a saturated or partially unsaturated heterocycle, an aryl, an heteroaryl or a fused aryl comprising 0-5 heteroatoms selected independently from nitrogen, oxygen or sulfur.

Another embodiment of present disclosure relates to enantiomerically and diastereomerically enriched HHPMs of general formula V, wherein Rₓ is H.

Another embodiment of present disclosure relates to enantiomerically and diastereomerically enriched HHPMs of general formula V, wherein Rₓ is H or alkyl.

Another embodiment of present disclosure relates to enantiomerically and diastereomerically enriched HHPMs of general formula V wherein,
- when R_{z} is a substituted aryl, said aryl is substituted with one or more groups R_{b} 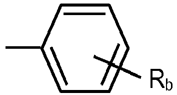 wherein R_{b} is independently selected from alkyl, alkoxy, halogen-alkyl, halogen, cyano, primary amine, secondary amines, tertiary amines or nitro; and
- when R_{z} is a fused aryl comprising 0-5 heteroatoms selected independently from nitrogen, oxygen or sulfur, said fused aryl is naphtyl.

One embodiment of present disclosure relates to enantiomerically and diastereomerically enriched HHPMs of general formula V wherein X is selected independently from -C(O)OR, -C(O)SR, -C(S)OR, -C(S)SR, -C(O)NH₂, -C(O)NHR, -C(O)N(R)₂, -C(O)NHOR, -C(O)NROR, -C(S)NH₂, -C(S)NHR, -C(S)N(R)₂, -C(S)NHOR, -C(S)NROR, -P(O)(OR)₂, -P(O)OR, -P(O)(SR)₂, -P(O)SR, -P(S)(SR)₂, -P(S)SR, -P(O)(NH₂)₂, -P(O)(NHR)₂, -P(O)[N(R)₂]₂, -P(S)(NH₂)₂, -P(S)(NHR)₂, -CN, -NO₂, -SO₂R, -SOR or -P(S)[N(R)₂]₂, and wherein R is alkyl.

Another embodiment of present disclosure relates to enantiomerically and diastereomerically enriched HHPMs of general formula V, wherein X is -C(O)OR.

Another embodiment of present disclosure relates to enantiomerically and diastereomerically enriched HHPMs of general formula V, wherein X is -C(O)OR and R is selected from a methyl or an ethyl group.

Another embodiment of present disclosure relates to enantiomerically and diastereomerically enriched HHPMs of general formula V, wherein X is -C(O)OR and R is ethyl.

One embodiment of present disclosure relates to enantiomerically and diastereomerically enriched HHPMs of general formula V, wherein Rₐ is alkyl.

Another embodiment of present disclosure relates to enantiomerically and diastereomerically enriched HHPMs of general formula V, wherein Rₐ is a methyl or an ethyl group.

Another embodiment of present disclosure relates to enantiomerically and diastereomerically enriched HHPMs of general formula V, wherein Rₐ is ethyl.

Another embodiment of present disclosure relates to enantiomerically and diastereomerically enriched HHPMs of general formula V, wherein R_{z} is phenyl or 4-fluorophenyl.

### EXAMPLES

### EXAMPLE 1: synthesis of α-ureidosulfones

The following α-ureidosulfones were prepared according to the method of Engberts et al. (Recl. Trav. Chim. Pays-Bas (1965) 84, 942-950) as disclosed in J.M. Saá et al. (c.f. Angew. Chem. Int. Ed. (2016), 55, 4312).

### 1-(Phenyl(tosyl)methyl)urea (1a):

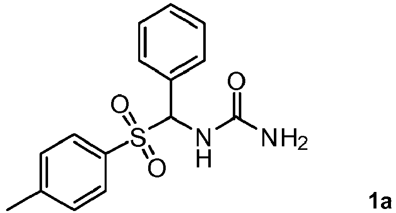

White solid, 80% yield; Mp 130°C (decomposition); ¹H NMR (300 MHz, DMSO-D6) δ: 2.39 (3H, s, CH₃), 5.79 (2H, brs, NH₂), 6.12 (1H, d, *J* = 10.6, C*H*NH), 7.38-7.46 (7H, m, 7xArH), 7.68-7.78 (3H, m, 2xArH, CHN*H*); ¹³C NMR (75.5 MHz, DMSO-D6) δ: 21.1 (CH₃), 73.1 (CH), 124.5, 128.2, 129.0, 129.1, 129.5 (ArCH), 131.6, 134.4, 144.4 (ArC), 156.2 (CO); HRMS (ESI⁺) Exact mass calc. for C₁₅H₁₆N₂NaO₃S [M+Na]⁺: 327.0779, found: 327.0774.

### 1-(p-Tolyl(tosyl)methyl)urea (1b):

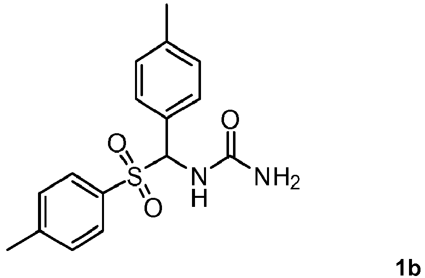

White solid, 98% yield; Mp 162°C (decomposition); ¹H NMR (300 MHz, DMSO-D6) δ: 2.33 (3H, s, CH₃), 2.40 (3H, s, CH₃), 5.75 (2H, br s, NH₂), 6.04 (1H, d, *J* = 10.7, C*H*NH), 7.22 (2H, d, *J* = 7.9, 2xArH), 7.33 (2H, d, *J* = 8.0, 2xArH), 7.40 (2H, d, *J* = 8.0, 2xArH), 7.63-7.75 (3H, m, 2xArH, CHN*H*); ¹³C NMR (75.5 MHz, DMSO-D6) δ: 20.8, 21.1 (CH₃), 72.9 (CH), 128.5 (ArC), 128.7, 129.0, 129.5 (ArCH), 134.4, 138.6, 144.3 (ArC), 156.1 (CO); HRMS (ESI⁺) Exact mass calc. for C₁₆H₁₈N₂NaO₃S [M+Na]⁺: 341.0936, found: 341.0930.

### 1-(Naphthalen-2-yl(tosyl)methyl)urea (1c):

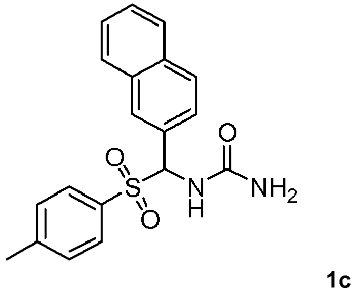

White solid, 91% yield; Mp 145°C (decomposition); ¹H NMR (300 MHz, DMSO-D6) δ: 2.39 (3H, s, CH₃), 5.88 (2H, Br s, NH₂), 6.35 (1H, d, *J* = 10.2, *CH*NH), 7.42 (2H, d, *J* = 7.2, 2 ×ArH), 7.57-7.68 (3H, m, 2×ArH, CHN*H*), 7.78 (2H, d, *J* = 7.5, 2xArH), 7.93-8.04 (5H, m, 5xArH); ¹³C NMR (75.5 MHz, DMSO-D6) δ: 21.2 (CH₃), 73.4 (CH), 126.4, 126.7, 127.0, 127.7, 127.8, 128.0, 128.8, 129.1 (ArC), 129.2 (ArCH), 132.4, 133.1, 134.4, 144.5 (ArC), 156.2 (CO); HRMS (ESI⁺) Exact mass calc. for C₁₉H₁₈N₂NaO₃S [M+Na]⁺: 377.0936, found: 377.0931.

### 1-((4-Fluorophenyl)(tosyl)methyl)urea (1e):

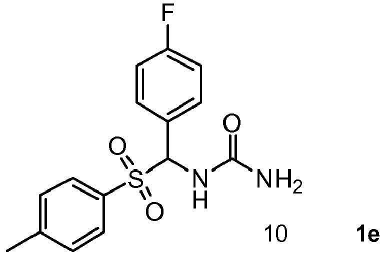

White solid, 98% yield; Mp 105°C (decomposition); ¹H NMR (300 MHz, DMSO-D6) δ: 2.38 (3H, s, CH₃), 5.78 (2H, br s, NH₂), 6.16 (1H, d, *J* = 10.5, CH), 7.23-7.54 (6H, m, 6xArH), 7.69-7.78 (3H, m, 2xArH, CHN*H*); ¹³C NMR (75.5 MHz, DMSO-D6) δ: 21.2 (CH₃), 72.4 (CH), 115.1, 115.4 (ArCH), 126.0 (ArC), 128.0, 129.1, 131.3, 131.4 (ArCH), 134.2, 144.5 (ArC), 156.2 (CO), 161.0, 164.3 (ArC); HRMS (ESI⁺) Exact mass calc. for C₁₅H₁₆FN₂O₃S [M+H]⁺: 323.0866, found: 323.0866.

### 1-((3-Fluorophenyl)(tosyl)methyl)urea (1f):

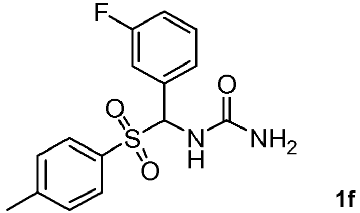

White solid, 81% yield; Mp 173°C (decomposition); ¹H NMR (300 MHz, DMSO-D6) δ: 2.40 (3H, s, CH₃), 5.80 (2H, brs, NH₂), 6.23 (1H, d, *J* = 10.8, CHNH), 7.23-7.53 (5H, m, 5xArH), 7.73 (2H, d, *J* = 8.1, 2xArH), 7.81 (1H, d, *J* = 10.6, CHN*H*); ¹³C NMR (75.5 MHz, DMSO-D6) δ: 21.2 (CH₃), 72.5 (CH), 115.9, 115.9, 116.2, 116.2, 124.5, 125.4, 129.1, 129.6, 130.2, 130.3 (ArCH), 134.1, 134.3, 134.4, 144.6 (ArC), 156.2 (CO), 159.9, 160.2, 163.4 (ArC); HRMS (ESI⁺) Exact mass calc. for C₁₅H₁₅FN₂NaO₃S [M+Na]⁺: 345.0685, found: 345.0679.

### 1-((2-Fluorophenyl)(tosyl)methyl)urea (1g):

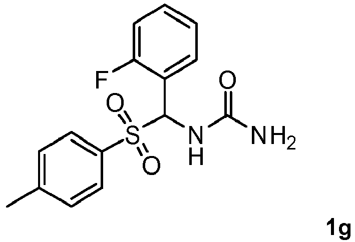

White solid, 95% yield; Mp 180°C (decomposition); ¹H NMR (300 MHz, DMSO-D6) δ: 2.40 (3H, s, CH₃), 5.89 (2H, br s, NH₂), 6.36 (1 H, d, *J* = 10.5, CH), 7.22-7.36 (2H, m, 2xArH), 7.41-7.55 (4H, m, 4xArH), 7.67 (2H, d, *J* = 8.1, 2xArH), 7.85 (2H, d, *J* = 10.5, CHN*H*); ¹³C NMR (75.5 MHz, DMSO-D6) δ: 22.1 (CH₃), 67.9 (CH), 116.3, 116.6 (ArCH), 120.2, 120.3 (ArC), 125.6, 129.9, 130.7, 131.0, 132.5, 132.6 (ArCH), 134.9, 145.8 (ArC), 157.1 (CO), 159.4, 162.7 (ArC): HRMS (ESI⁺) Exact mass calc. for C₁₅H₁₅FN₂NaO₃S [M+Na]⁺: 345.0685, found: 345.0680.

### 1-((2-Chlorophenyl)(tosyl)methyl)urea (1h):

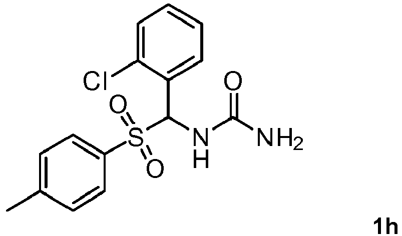

White solid, 86% yield; Mp 152°C (decomposition); ¹H NMR (300 MHz, DMSO-D6) δ: 2.41 (3H, s, CH₃), 5.83 (2H, br s, NH₂), 6.62 (1 H, d, *J* = 10.6, CH), 7.41-7.54 (5H, m, 5xArH), 7.56-7.61 (1 H, m, ArH), 7.67 (2H, d, *J* = 8.2, 2xArH), 7.87 (1 H, d, *J* = 10.6, CHN*H*); ¹³C NMR (75.5 MHz, DMSO-D6) δ: 21.2 (CH₃), 69.3 (CH), 127.5, 128.8, 129.4, 129.8, 129.9 (ArCH), 130.2 (ArC), 131.0 (ArCH), 134.2, 134.3, 144.8 (ArC), 156.1 (CO); HRMS (ESI⁺) Exact mass calc. for C₁₅H₁₅ClN₂NaO₃S [M+Na]⁺: 361.0390, found: 361.0377.

### 1-((2-Bromophenyl)(tosyl)methyl)urea (1i):

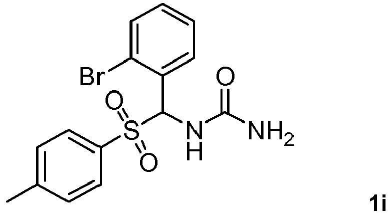

White solid, 78% yield; Mp 159°C (decomposition); ¹H NMR (300 MHz, DMSO-D6) δ: 2.39 (3H, s, CH₃), 5.87 (2H, brs, NH₂), 6.67 (1H, d, *J* = 10.2, CHNH), 7.44 (2H, d, *J* = 7.8, 2xArH), 7.75-7.82 (4H, m, 4xArH), 7.33-7.92 (8H, m, 8xArH), 7.93 (1 H, *J* = 10.2, CHNH); ¹³C NMR (75.5 MHz, DMSO-D6) δ: 21.2 (CH₃), 71.9 (CH), 125.3 (ArC), 128.0, 128.8, 129.8, 130.1, 131.3 (ArCH), 132.0 (ArC), 132.8 (ArCH), 134.4, 144.9 (ArC), 156.1 (CO); HRMS (ESI⁺) Exact mass calc. for C₁₅H₁₅BrN₂NaO₃S [M+Na]⁺: 404.9884, found: 404.9880.

### 1-((4-Cyanophenyl)(tosyl)methyl)urea (1j):

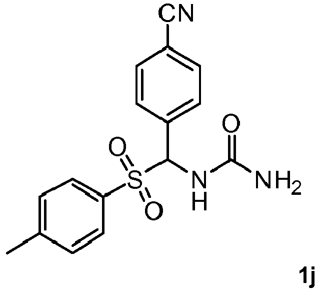

White solid, 78% yield; Mp 148°C (decomposition); ¹H NMR (300 MHz, DMSO-D6) δ: 2.39 (3H, s, CH₃), 5.81 (2H, br s, NH₂), 6.31 (1 H, d, *J* = 10.8, CH), 7.42 (2H, d, *J* = 7.8, 2xArH), 7.70-7.56 (4H, m, 4xArH), 7.84 -7.93 (3H, m, 2xArH, CHN*H*)); ¹³C NMR (75.5 MHz, DMSO-D6) δ: 21.2 (CH₃), 72.6 (CH), 111.9 (ArCCN), 118.5 (CN), 129.1, 129.6, 130.1, 132.1 (ArCH), 133.9, 137.0 , 144.8 (ArC), 156.1 (CO); HRMS (ESI⁺) Exact mass calc. for C₃₂H₃₀N₆NaO₆S₂ [2M+Na]⁺: 681.1566, found: 681.1566.

### 1-((4-nitrophenyl)(tosyl)methyl)urea (1k):

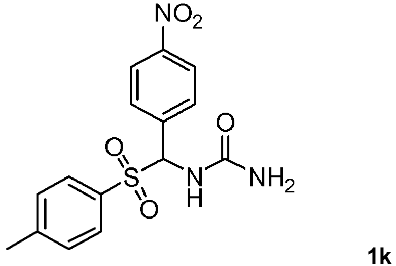

White solid, 92% yield; Mp 159°C (decomposition); ¹H NMR (300 MHz, DMSO-D6) δ: 2.41 (3H, s, CH₃), 5.83 (2H, brs, NH₂), 6.39 (1H, d, *J* = 10.8, CHNH), 7.44 (2H, d, *J* = 7.8, 2xArH), 7.75-7.82 (4H, m, 4xArH), 7.93 (1 H, d, *J* = 10.5, CHN*H*), 8.32 (2H, d, *J* = 8.7, 2×ArH); ¹³C NMR (75.5 MHz, DMSO-D6) δ: 21.2 (CH₃), 72.3 (CH), 123.3, 129.2, 129.7, 130.5 (ArCH), 133.9, 138.9, 144.9, 148.0 (ArC), 156.1 (CO); HRMS (ESI⁺) Exact mass calc. for C₁₅H₁₅N₃NaO₅S [M+Na]⁺: 372.0630, found: 372.0626.

### 1-((3-Nitrophenyl)(tosyl)methyl)urea (1l):

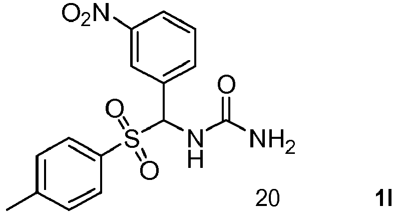

White solid, 85% yield; Mp 193°C (decomposition); ¹H NMR (300 MHz, DMSO-D6) δ: 2.40 (3H, s, CH₃), 5.85 (2H, brs, NH₂), 6.46 (1H, d, *J* = 10.5, CHNH), 7.43 (2H, d, *J* = 7.5, 2xArH), 7.70-7.81 (3H, m, 3xArH), 7.96-8.05 (2H, m, ArH, CHN*H*), 8.29 (1H, d, *J* = 7.8, ArH), 8.42 (1H, s, ArH); ¹³C NMR (75.5 MHz, DMSO-D6) δ: 21.2 (CH₃), 72.1 (CH), 123.5, 124.1, 129.2, 129.6, 129.8 (ArCH), 133.8, 134.0 (ArC), 136.1 (ArCH), 144.9, 147.7 (ArC), 156.14 (CO); HRMS (ESI⁺) Exact mass calc. for C₁₅H₁₅N₃NaO₅S [M+Na]⁺: 372.0630, found: 372.0626.

### 1-(Tosyl(4-(trifluoromethyl)phenyl)methyl)urea (1m):

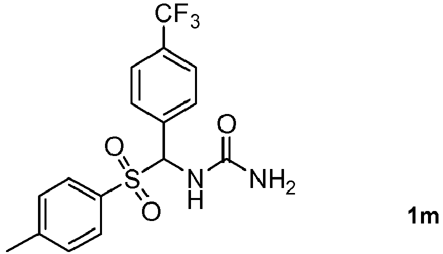

White solid, 89% yield; Mp 125°C (decomposition); ¹H NMR (300 MHz, DMSO-D6) δ: 2.40 (3H, s, CH₃), 5.84 (2H, brs, NH₂), 6.33 (1H, d, *J=* 10.2, CHNH), 7.43 (2H, d, *J=* 7.8, 2xArH), 7.72-7.94 (7H, m, 6xArH, CHN*H*)); ¹³C NMR (75.5 MHz, DMSO-D6) δ: falta 21.2 (CH₃), 72.6 (CH), 125.2, 129.3, 129.7, 130.1 (ArCH), 134.1, 136.3, 144.8, (ArC), 156.3 (CO), 163.2 (ArC); HRMS (ESI⁺) Exact mass calc. for C₁₆H₁₅F₃N₂NaO₃S [M+Na]⁺: 395.0653, found: 395.0650.

### 1-((4-Methoxyphenyl)(tosyl)methyl)urea (1n):

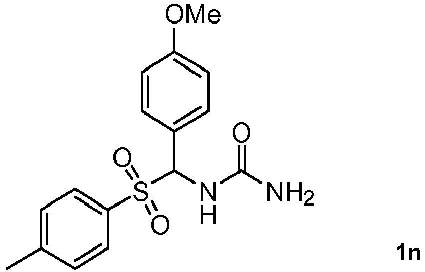

White solid, 90% yield; Mp 159°C (decomposition); ¹H NMR (300 MHz, DMSO-D6) δ: 2.39 (3H, s, CH₃Ph), 3.78 (3H, s, CH₃O), 5.75 (1H, br s, NH₂), 6.04 (1H, d, *J =* 10.6, CH), 6.98 (2H, d, *J* = 8.4, 2xArH), 7.32-7.45 (4H, m, 4xArH), 7.63-7.66 (3H, m, 2xArH, CHN*H*); ¹³C NMR (75.5 MHz, DMSO-D6) δ: 21.1 (CH₃Ph), 55.2 (CH₃O), 72.7 (CH), 113.6 (ArCH), 123.3 (ArC), 129.0, 129.4, 130.4 (ArCH), 134.5, 144.2, 156.1 (ArC), 159.9 (CO); HRMS (ESI⁺) Exact mass calc. for C₁₆H₁₈N₂NaO₄S [M+Na]⁺: 357.0885, found: 357.0871.

### 1-((3-Methoxyphenyl)(tosyl)methyl)urea (1o):

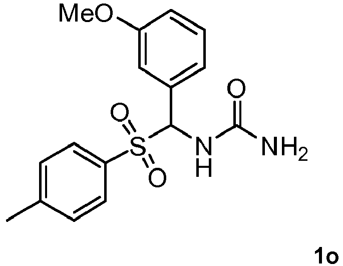

White solid, 96% yield; Mp 141°C (decomposition); ¹H NMR (300 MHz, DMSO-D6) δ: 2.40 (3H, s, CH₃Ph), 3.75 (3H, s, CH₃O), 5.79 (2H, br s, NH₂), 6.11 (1H, d, *J* = 10.8, CH), 6.97-7.05 (3H, m, 3xArH), 7.30- 7.42 (3H, m, 3xArH), 7.69-7.77 (3H, m, 2xArH, CHN*H*); ¹³C NMR (75.5 MHz, DMSO-D6) δ: 22.2 (CH₃Ph), 55.2 (CH₃O), 73.1 (CH), 114.5, 115.0, 121.4, 129.1, 129.3, 129.5 (ArCH), 133.1, 134.4, 141.4, 144.4, 156.2 (ArC), 159.0 (CO); HRMS (ESI⁺) Exact mass calc. for C₁₆H₁₈N₂NaO₄S [M+Na]⁺: 357.0885, found: 357.0879.

### 1-(Furan-2-yl(tosyl)methyl)urea (1p):

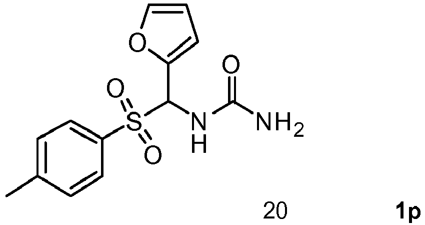

Light Brown solid, 75% yield; Mp 136°C (decomposition); ¹H NMR (300 MHz, DMSO-D6) δ: 2.39 (3H, s, CH₃), 5.87 (2H, br s, NH₂), 6.13 (1H, d, *J* = 9.9, CH), 6.50-6.56 (2H, m, 2xCHFur), 7.40 (2H, d, *J* = 7.2 , 2xArH), 7.56-7.73 (4H, m, CHN*H*, CHFur, 2xArH); ¹³C NMR (75.5 MHz, DMSO-D6) δ: 21.2 (CH₃), 68.4 (CH), 111.1, 111.6 (FurCH), 129.0, 129.6 (ArCH), 133.9 (ArC), 144.4 (FurCH), 144.6, 145.1 (FurC, ArC), 156.0 (CO); HRMS (ESI⁺) Exact mass calc. for C₂₆H₂₈N₄NaO₈S₂ [2M+Na]⁺: 611.1246, found: 611.1243.

### 1-(Thiophen-2-yl(tosyl)methyl)urea (1q):

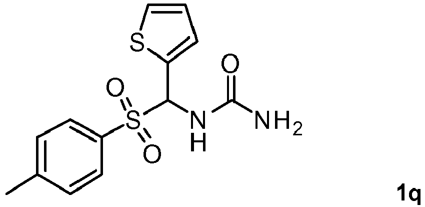

White solid, 69% yield; Mp 132°C (decomposition); ¹H NMR (300 MHz, DMSO-D6) δ: 2.38 (3H, s, CH₃), 5.84 (2H, br s, NH₂), 6.34 (1 H, d, *J* = 10.5, CH), 7.09 (1 H, d, *J* = 4.2, CHThio), 7.23 (1 H, d, *J =* 2.1, CHThio), 7.40 (2H, d, *J =* 7.8, 2xArH), 7.62-7.66 (4H, m, 1×CHThio, 2xArH, CHN*H*) ); ¹³C NMR (75.5 MHz, DMSO-D6) δ: 21.2 (CH₃), 69.5 (CH), 127.1, 128.0 (ThioCH), 129.1, 129.6 (ArCH), 133.6, 133.8 (ArC), 144.6 (ThioC), 156.0 (CO); HRMS (ESI⁺) Exact mass calc. for C13H14N2O3S2 [M+Na]⁺: 333.0344, found: 333.0339.

### 1-((3,4-Difluorophenyl)(tosyl)methyl)urea (1s):

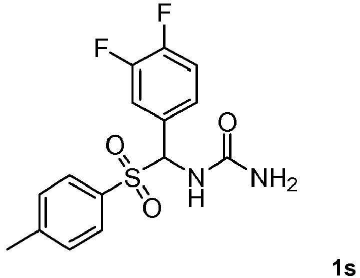

White solid, 74% yield; Mp 172°C (decomposition); ¹H NMR (300 MHz, DMSO-D6) δ: 2.41 (3H, s, CH₃), 5.79 (2H, brs, NH₂), 6.26 (1H, d, *J* = 10.5, CHNH), 7.35-7.64 (5H, m, 5xArH), 7.74 (2H, d, *J =* 8.1, 2xArH), 7.82 (1H, d, *J =* 10.5, CHN*H*); ¹³C NMR (75.5 MHz, DMSO-D6) δ: 21.2 (CH₃), 72.0 (CH), 117.4, 117.6, 118.3, 118.5, 126.4, 129.2 (ArCH), 129.5 (ArC), 129.6 (ArCH), 134.0, 144.7 (ArC), 156.1 (CO), 159.9, 160.2, 163.4 (ArC); HRMS (ESI⁺) Exact mass calc. for C₁₅H₁₄F₂N₂NaO₃S [M+Na]⁺: 363.0591, found: 363.0587.

### 1-(Tosyl(3-(trifluoromethyl)phenyl)methyl)urea (1u):

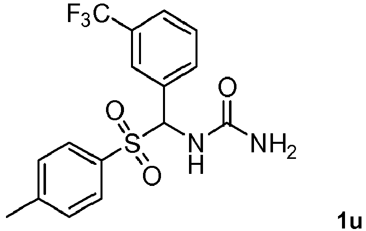

White solid, 83% yield; Mp 156°C (decomposition); ¹H NMR (300 MHz, DMSO-D6) δ: 2.41 (3H, s, CH₃), 5.81 (2H, br s, NH₂), 6.38 (1 H, d, *J* = 10.8, CHNH), 7.04 (7.43 (2H, d, *J* = 7.8, 2xArH), 7.72-7.94 (7H, m, 6xArH, CHN*H*)); ¹³C NMR (75.5 MHz, DMSO-D6) δ: 21.2 (CH₃), 72.5 (CH), 123.5, 124.1, 129.2, 129.6, 129.8 (ArCH), 133.8, 134.0 (ArC), 136.1 (ArCH), 144.9, 147.7 (ArC), 157.9 (CO); HRMS (ESI⁺) Exact mass calc. for C₁₆H₁₅F₃N₂NaO₃S [M+Na]⁺: 395.0653, found: 395.0652.

### EXAMPLE 2: "one pot" enantioselective synthesis of DHPMs from α-ureidosulfones

To a suspension of an α-ureidosulfone (0.2 mmoles) of Example 1 in dichloromethane (2 ml), cooled down to 0°C, N,N'-diisopropylethylamine (0.8 mmoles, 0.14 ml, 4 equivalents) is added dropwise, followed by a dichloromethane solution (2 ml) of the organocatalysts of formula VI or VII: 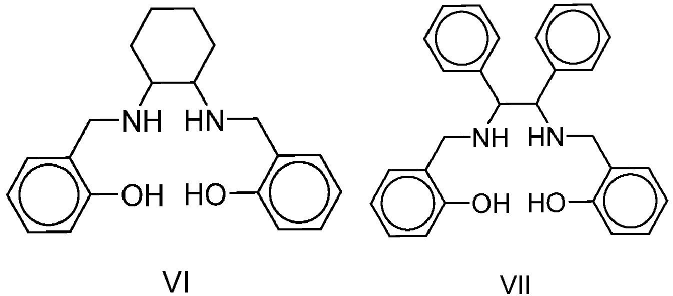 diastereomerically and enantiomerically enriched with one *(R, R)* or (S, S) stereoisomer, (0.02 mmoles, 0.1 equivalents) and the corresponding methyl or ethyl acetoacetate (0.21 mmoles). Stirring at 0°C is maintained for 20 hours. After this, 5M HCl (2ml) is added to the two-phase mixture and stirred at room temperature for another 5 hours. Brine (17 ml) is added, and the mixture is extracted three times with ethyl acetate (3x15ml). The combined organic phases are dried with anhydrous MgSO₄, then filtered and the solvent rotoevaporated under vacuum. The crude solid material is then washed with diethyl ether and hexane and checked in HPLC for enantiomeric purity (typically using a Chiralpak OD-H chiral column from Daicel) prior to recrystallization.

The organocatalysts of formula VI employed are enantiomerically and diastereomerically enriched species which have been prepared, as disclosed in J.M. Saá et al. (c.f. Angew. Chem. Int. Ed. (2016), 55, 4312), starting from a commercial diamine precursor: (1*R*,2*R*)-1,2-cyclohexanediamine, which is sold with an optical purity of 99% ee (Sigma Aldrich), and (1*S*,2*S*)-1,2-cyclohexanediamine which is sold with an optical purity of 99% ee (Fluorochem).

In an analogous manner, the organocatalysts of formula VII employed are enantiomerically and diastereomerically enriched species which have been also prepared, as disclosed in J.M. Saá et al. (c.f. Angew. Chem. Int. Ed. (2016), 55, 4312), starting from a commercial diamine precursor: (1 R,2R)-1,2-diphenylethylene-1,2-diamine, which is sold with an optical purity of 99% ee (Sigma Aldrich), and (1*S*,2*S*)-1,2-diphenylethylene-1,2-diamine which is sold with an optical purity of 99% ee (Apollo Scientific).

The procedure to obtain said organocatalysts of formula VI and VII was therefore the following:

To a solution of salicyladehyde (1 mmol) in methanol (1 ml) was added dropwise a solution of the commercially available diamine precursor (0.5 mmol) in methanol (1.5 ml) and the resulting solution was stirred during 6 hours. After cooling at 0°C, NaBH₄ (4 mmol) was added in five portions and the corresponding mixture was stirred overnight at room temperature. Then the suspension was poured into a saturated NH₄Cl solution (100 ml) and extracted with dichloromethane (3×30 ml). The combined organic layers were dried over MgSO₄ and evaporated under reduced pressure to obtain the product as a light brown solid which can be recrystallized in hexane/dichloromethane.

The following enantiomerically enriched DHPMs **2a** to **2u** were synthesized according to this example (the (*R*) or (*S*) configuration of the DHPM synthesized in enantiomeric excess is featured for each case):

### Ethyl (R)-6-methyl-2-oxo-4-phenyl-1,2,3,4-tetrahydropyrimidine-5-carboxylate [(R)-2a]:

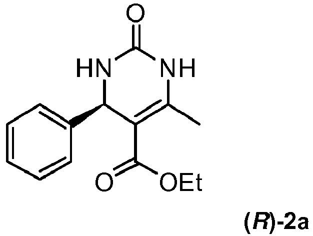

White solid, 96% yield; [α]D²⁵ = -58 (c = 0.5, 94% ee, MeOH); Mp 203°C (Lit. 201-203°C); ¹H NMR (300 MHz, DMSO-D6) δ: 1.09 (3H, t, *J* = 7.1, CH₂C*H*₃), 2.25 (3H, s, =CCH₃), 3.98 (2H, q, *J =* 7.1, CH₂), 7.21-7.35 (5H, m, 5xArH), 7.75 (1 H, s, CHN*H*), 9.20 (1 H, s, NHC=); ¹³C NMR (75.5 MHz, DMSO-D6) δ: 14.1 (*C*H₃CH₂),17.8 (=C*C*H₃), 54.0 (CHNH), 59.2 (CH₂), 99.3 (COC=), 126.3, 127.3, 128.4 (ArCH), 144.9, 148.4 (=*C*CH₃, ArC), 152.2 (NHCONH), 165.4 (COO).

Organocatalyst of formula (*R,R*)-VI used. The enantiomeric excess was determined by HPLC analysis (Chiralpak OD-H, hexane/*i*-PrOH = 8:2, 0.5 ml/min, 254 nm, *t*ᵣ (minor) = 16.48 min, *t*ᵣ (major) = 20.23 min, 97% ee.

### Ethyl (S)-6-methyl-2-oxo-4-phenyl-1,2,3,4-tetrahydropyrimidine-5-carboxylate [(S)-2a]:

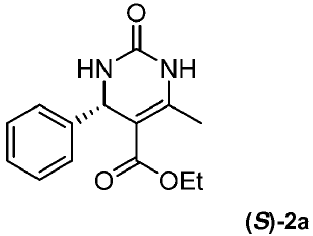

White solid, 98% yield; Mp 203°C (Lit. 201-203°C); ¹H NMR (300 MHz, DMSO-D6) δ: 1.09 (3H, t, *J* = 7.1, CH₂C*H*₃), 2.25 (3H, s, =CCH₃), 3.98 (2H, q, *J* = 7.1, CH₂), 7.21-7.35 (5H, m, 5xArH), 7.75 (1 H, s, CHN*H*), 9.20 (1 H, s, NHC=); ¹³C NMR (75.5 MHz, DMSO-D6) δ: 14.1 (*C*H₃CH₂), 17.8 (=C*C*H₃), 54.0 (CHNH), 59.2 (CH₂), 99.3 (CO*C*=), 126.3, 127.3, 128.4 (ArCH), 144.9, 148.4 (=*C*CH₃, ArC), 152.2 (NHCONH), 165.4 (COO).

Organocatalyst of formula (*S*,*S*)-VI used. The enantiomeric excess was determined by HPLC analysis (Chiralpak OD-H, hexane/*i*-PrOH = 8:2, 0.5 ml/min, 254 nm, *t*ᵣ (major) = 16.90 min, *t*ᵣ (minor) = 21.33 min, 99% ee.

### Ethyl (R)-6-methyl-2-oxo-4-(p-tolyl)-1,2,3,4-tetrahydropyrimidine-5-carboxylate [(R)-2b]:

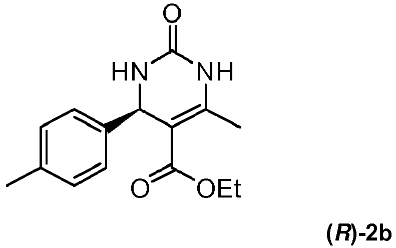

White solid, 80% yield, Mp 222°C (Lit. 229-230°C); ¹H NMR (300 MHz, DMSO-D6) δ: 1.09 (3H, t, *J* = 7.1, CH₂C*H*₃), 2.23 (3H, s, CH₃), 2.25 (3H, s, CH₃), 3.97 (2H, q, *J* = 7.1, CH₂), 5.10 (1H, d, *J=* 3.3, C*H*NH), 7.11 (4H, s, 4xArH), 7.68 (1H, s, CHN*H*), 9.15 (1H, s, NHC=); ¹³C NMR (75.5 MHz, DMSO-D6) δ: 14.1 (*C*H₃CH₂), 17.8 (=C*C*H₃), 20.6 (PhCH₃), 53.6 (CHNH), 59.2 (CH₂), 99.4 (CO*C*=), 126.1, 128.9 (ArCH), 136.4 (Ar*C*CH₃), 142.0 (ArC), 148.1 (=*C*CH₃), 152.2 (NHCONH), 165.4 (COO).

Organocatalyst of formula *(R,R)*-VI used. The enantiomeric excess was determined by HPLC analysis (Chiralpak OD-H, hexane/*i*-PrOH = 8:2, 0.5 ml/min, 254 nm, *t*ᵣ (minor) = 15.47 min, *t*ᵣ (major) = 20.89 min, 97% ee.

### Ethyl (R)-6-methyl-4-(naphthalen-2-yl)-2-oxo-1,2,3,4-tetrahydropyrimidine-5-carboxylate [(R)-2c]:

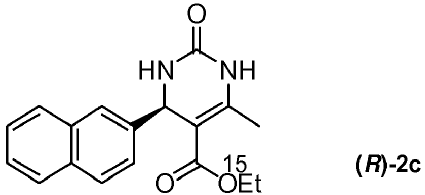

White solid, 79% yield; Mp 201°C (Lit. 210-212°C); ¹H NMR (300 MHz, DMSO-D6) δ: 1.08 (3H, t, *J* = 7.1, CH₂C*H*₃), 2.33 (3H, s, =CCH₃), 3.98 (2H, q, *J* = 7.2, CH₂), 5.37 (1H, d, *J* = 3.0, CH), 7.46-7.53 (3H, m, 3xArH), 7.71 (1H, s, CHN*H*), 7.85-7.92 (4H, m, 4xArH), 9.31 (1H, s, NHC=); ¹³C NMR (75.5 MHz, DMSO-D6) δ: 14.1 (*C*H₃CH₂), 17.9 (=C*C*H₃), 54.0 (CHNH), 59.2 (CH₂), 99.1 (CO*C*=), 124.6, 125.0, 125.9, 126.3, 127.5, 127.8, 128.4 (ArCH), 132.4, 132.7. 148.7 (=*C*CH₃, ArC), 152.2 (NHCONH), 165.4 (COO).

Organocatalyst of formula (*R,R*)-VI used. The enantiomeric excess was determined by HPLC analysis (Chiralpak OD-H, hexane/*i*-PrOH = 8:2, 0.5 ml/min, 280 nm, *t*ᵣ (minor) = 24.12 min, *t*ᵣ (major) = 30.37 min, 95% ee.

### Ethyl (R)-6-ethyl-2-oxo-4-phenyl-1,2,3,4-tetrahydropyrimidine-5-carboxylate [(R)-2d]:

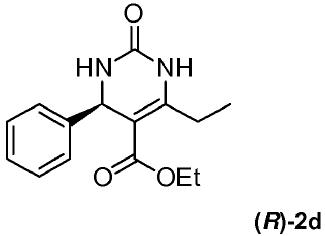

White solid, 97% yield; Mp 121°C (Lit. 128-131°C); ¹H NMR (300 MHz, DMSO-D6) δ: 1.06-1.15 (6H, m, 2×CH₂C*H*₃), 2.54-2.78 (2H, m, =CCH₂), 3.98 (2H, q, *J =* 7.2, CH₂), 5.15 (1 H, d, *J* = 3.3*,* CH), 7.19-7.35 (5H, m, 5xArH), 7.75 (1 H, s, CHN*H*), 9.23 (1 H, s, NHC=); ¹³C NMR (75.5 MHz, DMSO-D6) δ: 13.0, 14.0 (CH₃), 24.1 (=C*C*H₂), 54.0 (CHNH), 59.2 (OCH₂), 98.5 (CO*C*=), 126.3, 127.3, 128.4 (ArCH), 144.9, 152.5, 153.9 (=*C*CH₃, ArC, NHCONH), 165.0 (COO).

Organocatalyst of formula (*R,R*)-VI used. The enantiomeric excess was determined by HPLC analysis (Chiralpak OD-H, hexane/*i*-PrOH = 8:2, 0.5 ml/min, 254 nm, *t*ᵣ (minor) = 15.61 min, *t*ᵣ (major) = 21.73 min, 92% ee.

### Ethyl (R)-4-(4-fluorophenyl)-6-methyl-2-oxo-1,2,3,4-tetrahydropyrimidine-5-carboxylate[(R)-2e]:

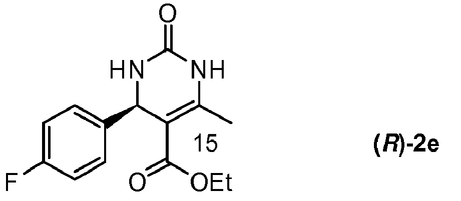

White solid, 92% yield; Mp 190 °C (Lit. 190-192°C); ¹H NMR (300 MHz, DMSO-D6) δ: 1.07 (3H, t, *J* = 7.2, CH₂C*H*₃), 2.24 (3H, s, =CCH₃), 3.97 (2H, q, *J* = 7.2, CH₂), 5.14 (1H, d, *J* = 3.3, C*H*NH), 7.10-7.17 (2H, m, 2xArH), 7.23-7.76 (2H, m, 2xArH), 7.75 (1 H, s, CHN*H*), 9.22 (1 H, s, NHC=); ¹³C NMR (75.5 MHz, DMSO-D6) δ: 14.1 (*C*H₃CH₂), 17.8 (=C*C*H₃), 53.4 (CHNH), 59.2 (CH₂), 99.2 (CO*C*=), 115.3, 128.2 (ArCH), 141.1 (ArC), 148.5 (=*C*CH₃), 152.0 (NHCONH), 165.3 (COO).

Organocatalyst of formula (*R,R*)-VI used. The enantiomeric excess was determined by HPLC analysis (Chiralpak OD-H, hexane/*i*-PrOH = 8:2, 0.5 ml/min, 254 nm, *t*ᵣ (minor) = 14.67 min, *t*ᵣ (major) = 19.56 min, 94% ee.

### Ethyl (S)-4-(4-fluorophenyl)-6-methyl-2-oxo-1,2,3,4-tetrahydropyrimidine-5-carboxylate [(S)-2e]:

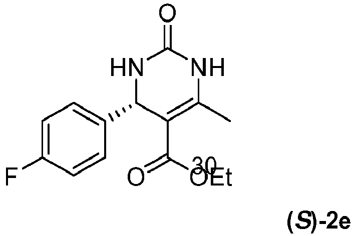

White solid, 94% yield; Mp 190 °C (Lit. 190-192°C); ¹H NMR (300 MHz, DMSO-D6) δ: 1.07 (3H, t, *J* = 7.2, CH₂C*H*₃), 2.24 (3H, s, =CCH₃), 3.97 (2H, q, *J =* 7.2, CH₂), 5.14 (1 H, d, *J =* 3.3, C*H*NH), 7.10-7.17 (2H, m, 2xArH), 7.23-7.76 (2H, m, 2xArH), 7.75 (1H, s, CHN*H*), 9.22 (1 H, s, NHC=); ¹³C NMR (75.5 MHz, DMSO-D6) δ: 14.1 (CH₃CH₂), 17.8 (=C*C*H₃), 53.4 (CHNH), 59.2 (CH₂), 99.2 (CO*C*=), 115.3, 128.2 (ArCH), 141.1 (ArC), 148.5 (=*C*CH₃), 152.0 (NHCONH), 165.3 (COO).

Organocatalyst of formula (*S,S*)-VI used. The enantiomeric excess was determined by HPLC analysis (Chiralpak OD-H, hexane/*i*-PrOH = 8:2, 0.5 ml/min, 254 nm, *t*ᵣ (major) = 15.41 min, *t*ᵣ (minor) = 21.26 min, 97% ee.

### Ethyl (R)-4-(3-fluorophenyl)-6-methyl-2-oxo-1,2,3,4-tetrahydropyrimidine-5-carboxylate [(R)-2f]:

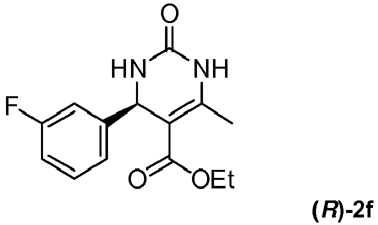

White solid, 99% yield; Mp 204°C (Lit. 212-213°C); ¹H NMR (300 MHz, DMSO-D6) δ: 1.10 (3H, t, *J* = 7.2, CH₂C*H*₃), 2.29 (3H, s, =CCH₃), 3.97-4.05 (2H, m, CH₂), 5.21 (1 H, d, *J* = 3.0, CH), 7.02-7.14 (3H, m, 3xArH), 7.34-7.42 (1H, m, ArH), 7.85 (1H, s, CHN*H*), 9.32 (1H, s, NHC=); ¹³C NMR (75.5 MHz, DMSO-D6) δ: 14.1 (CH₃CH₂), 17.9 (=C*C*H₃), 53.7 (CHNH), 59.4 (CH₂), 98.8 (CO*C*=), 112.9, 113.2, 114.0, 114.3, 122.3, 122.3 130.5, 130.6 (ArCH), 147.7, 147.8, 149.0, 152.2, 160.6, 163.8 (=*C*CH₃, ArC, NHCONH), 165.3 (COO).

Organocatalyst of formula (*R,R*)-VI used. The enantiomeric excess was determined by HPLC analysis (Chiralpak OD-H, hexane/*i*-PrOH = 8:2, 0.5 ml/min, 280 nm, *t*ᵣ (minor) = 15.89 min, *t*ᵣ (major) = 18.77 min, 98% ee.

### Ethyl (S)-4-(2-fluorophenyl)-6-methyl-2-oxo-1,2,3,4-tetrahydropyrimidine-5-carboxylate [(S)-2g]:

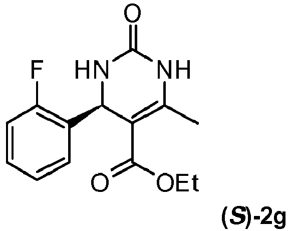

White solid, 93% yield; Mp 228°C (Lit. 235-237°C); ¹H NMR (300 MHz, DMSO-D6) δ: 1.03 (3H, t, *J* = 7.1, CH₂C*H*₃), 2.26 (3H, s, =CCH₃), 3.91 (2H, q, *J* = 7.1, CH₂), 5.45 (1H, d, *J* = 3.0, C*H*NH), 7.10-7.18 (2H, m, ArH), 7.23-7.33 (2H, m, ArH), 7.69 (1H, s, CHN*H*), 9.25 (1H, s, NHC=); ¹³C NMR (75.5 MHz, DMSO-D6) δ: 13.9 (*C*H₃CH₂), 17.7 (=C*C*H₃), 48.6 (CHNH), 59.1 (CH₂), 97.4 (CO*C*=), 115.3, 115.6, 124.5, 124.6, 128.8, 128.9, 129.3, 129.4 (ArCH), 149.0, 151.2 (=*C*CH₃, NHCONH), 157.7, 161.0 (ArC), 165.0 (COO).

Organocatalyst of formula (*R*,*R*)-VI used. The enantiomeric excess was determined by HPLC analysis (Chiralpak OD-H, hexane/*i*-PrOH = 8:2, 0.5 ml/min, 280 nm, *t*ᵣ (minor) = 16.44 min, *t*ᵣ (major) = 20.62 min, 96% ee.

### Ethyl (S)-4-(2-chlorophenyl)-6-methyl-2-oxo-1,2,3,4-tetrahydropyrimidine-5-carboxylate [(S)-2h]:

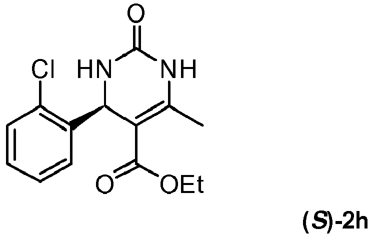

White solid, 98% yield; Mp 218 °C (Lit. 222-224 °C); ¹H NMR (300 MHz, DMSO-D6) δ: 0.98 (3H, t, *J* = 7.1, CH₂C*H*₃), 2.29 (3H, s, =CCH₃), 3.88 (2H, q, *J* = 7.1, CH₂), 5.63 (1H, d, *J* = 3.0, C*H*NH), 7.23-7.32 (3H, m, 3xArH), 7.37-7.41 (1H, m, ArH), 7.69 (1H, s, CHN*H*), 9.26 (1H, s, NHC=); ¹³C NMR (75.5 MHz, DMSO-D6) δ: 13.9 (*C*H₃CH₂), 17.7 (=C*C*H₃), 51.5 (CHNH), 59.1 (CH₂), 97.9 (CO*C*=), 127.8, 128.8, 129.1, 129.4 (ArCH), 131.7 (ArCI), 141.7 (ArC), 149.3 (=*C*CH₃), 151.4 (NHCONH), 165.0 (COO).

Organocatalyst of formula (*R,R*)-VI used. The enantiomeric excess was determined by HPLC analysis (Chiralpak OD-H, hexane/*i*-PrOH = 8:2, 0.5 ml/min, 300 nm, *t*ᵣ (minor) = 16.80 min, *t*ᵣ (major) = 21.12 min, 95% ee.

### Ethyl (S)-4-(2-bromophenyl)-6-methyl-2-oxo-1,2,3,4-tetrahydropyrimidine-5-carboxylate [(S)-2i]:

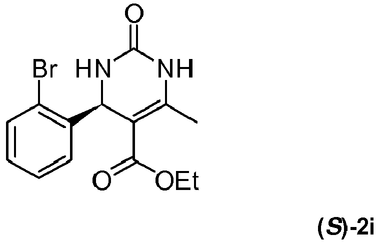

White solid, 97% yield; Mp 204°C (Lit. 206-208°C); ¹H NMR (300 MHz, DMSO-D6) δ: 0.99 (3H, t, *J* = 7.2, CH₂C*H*₃), 2.33 (3H, s, =CCH₃), 3.90 (2H, q, *J =* 7.1, CH₂), 5.64 (1 H, d, *J =* 2.4, CH), 7.14-7.21 (1 H, m, ArH), 7.31-7.39 (2H, m, 2xArH), 7.57 (1 H, d, *J* = 7.8, ArH), 7.73 (1 H, s, CHN*H*), 9.34 (1 H, s, NHC=); ¹³C NMR (75.5 MHz, DMSO-D6) δ: 14.0 (*C*H₃CH₂), 17.7 (=C*C*H₃), 54.1 (CHNH), 59.1 (CH₂), 98.3 (CO*C*=), 122.3 (ArC), 128.5, 128.8, 129.4, 132.6 (ArCH), 143.4, 149.3 (=*C*CH₃, ArC), 151.4 (NHCONH), 165.0 (COO).

Organocatalyst of formula (*R,R*)-VI used. The enantiomeric excess was determined by HPLC analysis (Chiralpak OD-H, hexane/*i*-PrOH = 8:2, 0.5 ml/min, 280 nm, *t*ᵣ (minor) = 17.19 min, *t*ᵣ (major) = 22.20 min, 96% ee.

### Ethyl (R)-4-(4-cyanophenyl)-6-methyl-2-oxo-1,2,3,4-tetrahydropyrimidine-5-carboxylate [(R)-2j]:

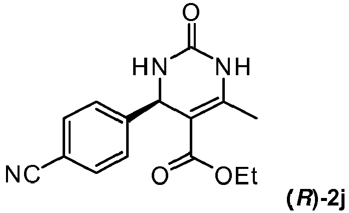

White solid, 82% yield, Mp 181°C (Lit. 180-182°C); ¹H NMR (300 MHz, DMSO-D6) δ: 1.08 (3H, t, *J* = 7.2, CH₂C*H*₃), 2.26 (3H, s, =CCH₃), 3.98 (2H, q, *J* = 7.1, C*H*₂CH₃), 5.21 (1 H, d, *J* = 3.0, C*H*NH), 7.42 (2H, d, *J* = 8.4, 2xArH), 7.82 (2H, d, *J* = 8.4, 2xArH), 7.86 (1 H, s, CHN*H*), 9.33 (1 H, s, NHC=); ¹³C NMR (75.5 MHz, DMSO-D6) δ: 14.1 (CH₂*C*H₃), 17.9 (=C*C*H₃), 53.88 (CHNH), 59.4 (CH₂), 98.2 (COC=), 110.1 (ArC), 118.8 (CN), 127.4, 132.6 (ArCH), 149.3, 150.0 (=*C*CH₃, ArC), 151.8 (CONH), 165.1 (COO).

Organocatalyst of formula (*R,R*)-VI used. The enantiomeric excess was determined by HPLC analysis (Chiralpak OD-H, hexane/*i*-PrOH = 8:2, 0.5 ml/min, 300 nm, *t*ᵣ (minor) = 24.36 min, *t*ᵣ (major) = 31.73 min, 84% ee.

### Ethyl (R)-6-methyl-4-(4-nitrophenyl)-2-oxo-1,2,3,4-tetrahydropyrimidine-5-carboxylate [(R)-2k]:

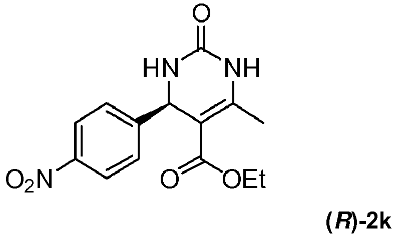

White solid, 98% yield; Mp 202°C (Lit. 206-208°C); ¹H NMR (300 MHz, DMSO-D6) δ: 1.11 (3H, t, *J* = 7.1, CH₂C*H*₃), 2.29 (3H, s, =CCH₃), 4.00 (2H, q, *J* = 7.1, CH₂), 5.30 (1 H, d, *J* = 3.0, CH), 7.53 (2H, d, *J* = 8.4, 2xArH), 7.92 (1 H, s, CHN*H*), 8.23 (2H, d, *J* = 8.1, 2xArH), 9.38 (1 H, s, NHC=); ¹³C NMR (75.5 MHz, DMSO-D6) δ: 14.1 (*C*H₃CH₂), 17.9 (=C*C*H₃), 53.7 (CHNH), 59.4 (CH₂), 98.2 (CO*C*=), 123.8, 127.7 (ArCH), 146.7, 149.4, 151.8, 152.0 (=*C*CH₃, ArC, NHCONH), 165.1 (COO).

Organocatalyst of formula (*R,R*)-VII used. The enantiomeric excess was determined by HPLC analysis (Chiralpak OD-H, hexane/*i*-PrOH = 8:2, 0.5 ml/min, 254 nm, *t*ᵣ (minor) = 26.02 min, *t*ᵣ (major) = 30.76 min, 68% ee.

### Ethyl (R)-6-methyl-4-(3-nitrophenyl)-2-oxo-1,2,3,4-tetrahydropyrimidine-5-carboxylate [(R)-2l]:

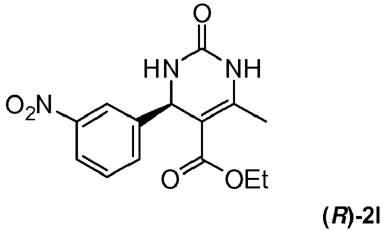

White solid, 82% yield; Mp 195°C (Lit. 205-206°C); ¹H NMR (300 MHz, DMSO-D6) δ: 1.11 (3H, t, *J* = 6.9, CH₂C*H*₃), 2.30 (3H, s, =CCH₃), 3.96-4.06 (2H, m, CH₂), 5.34 (1 H, d, *J* = 3.0, CH), 7.64-7.75 (2H, m, 2xArH), 7.94 (1H, s, CHN*H*), 8.11-8.17 (2H, m, 2xArH), 9.41 (1H, s, NHC=); ¹³C NMR (75.5 MHz, DMSO-D6) δ: 14.1 (*C*H₃CH₂), 17.9 (=C*C*H₃), 53.6 (CHNH), 59.4 (CH₂), 98.4 (CO*C*=), 121.1, 122.4, 130.3, 133.1 (ArCH), 147.0, 147.8, 149.5, 151.9 (=*C*CH₃, ArC, NHCONH), 165.1 (COO).

Organocatalyst of formula (*R,R*)-VI used. The enantiomeric excess was determined by HPLC analysis (Chiralpak OD-H, hexane/*i*-PrOH = 8:2, 0.5 ml/min, 300 nm, *t*ᵣ (minor) = 26.45 min, *t*ᵣ (major) = 28.11 min, 77% ee.

### Ethyl (R)-6-methy)-2-oxo-4-(4-(trifluoromethyl)phenyl)-1,2,3,4-tetrahydropyrimidine-5-carboxylate [(R)-2m]:

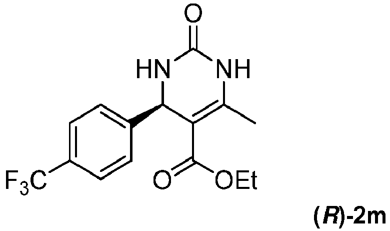

White solid, 87% yield; Mp 169°C (Lit. 173-175°C); ¹H NMR (300 MHz, DMSO-D6) δ: 1.10 (3H, t, *J =* 7.1, CH₂C*H*₃), 2.28 (3H, s, =CCH₃), 4.00 (2H, q, *J =* 7.1, CH₂), 5.26 (1 H, d, *J =* 2.4, CH), 7.48 (2H, d, *J =* 7.8, 2xArH), 7.72 (2H, d, *J =* 8.1, 2xArH), 7.88 (1 H, s, CHN*H*), 9.34 (1 H, s, NHC=); ¹³C NMR (75.5 MHz, DMSO-D6) δ: 14.1 (*C*H₃CH₂),17.9 (=C*C*H₃), 53.8 (CHNH), 59.4 (CH₂), 98.6 (CO*C*=), 125.0126.3, 127.3, 128.4 (ArCH), 149.1, 149.3, 152.0, 157.8 (=*C*CH₃, ArC, NHCONH), 165.2 (COO).

Organocatalyst of formula (*R,R*)-VII used. The enantiomeric excess was determined by HPLC analysis (Chiralpak OD-H, hexane/*i*-PrOH = 8:2, 0.5 ml/min, 280 nm, *tᵣ* (minor) = 19.84 min, *tᵣ* (major) = 26.62 min, 82% ee.

### Ethyl (R)-4-(4-methoxyphenyl)-6-methyl-2-oxo-1,2,3,4-tetrahydropyrimidine-5-carboxylate [(R)-2n]:

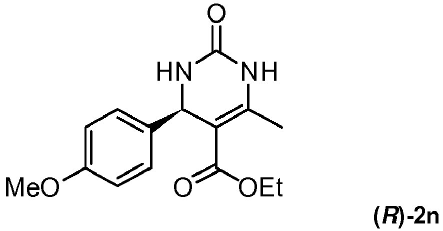

White solid, 81% yield; Mp 203°C (Lit. 199-201°C); ¹H NMR (300 MHz, DMSO-D6) δ: 1.09 (3H, t, *J* = 7.1, CH₂C*H*₃), 2.23 (3H, s, =CCH₃), 3.71 (3H, s, OCH₃), 3.97 (2H, q, *J* = 7.1, C*H*₂CH₃), 5.08 (1H, d, *J =* 3.0, CHNH), 6.87 (2H, d, *J =* 8.5, 2xArH), 7.14 (2H, d, *J =* 8.5, 2xArH), 7.66 (1H, s, CHN*H*), 9.14 (1H, s, NHC=); ¹³C NMR (75.5 MHz, DMSO-D6) δ: 14.1 (CH₂CH₃), 17.8 (=C*C*H₃), 53.5, 55.1 (CHNH, CH₃O), 59.2 (CH₂), 99.8 (CO*C*=), 127.4, 133.7 (ArCH), 137.2 (ArC), 148.2, 152.2 (=*C*CH₃, CONH), 158.5 (ArC), 165.4 (COO).

Organocatalyst of formula *(R,R)-VI* used. The enantiomeric excess was determined by HPLC analysis (Chiralpak OD-H, hexane/*i*-PrOH = 8:2, 0.5 ml/min, 254 nm, *tᵣ* (minor) = 20.86 min, *tᵣ* (major) = 28.00 min, 93% ee.

### Ethyl (R)-4-(3-methoxyphenyl)-6-methyl-2-oxo-1,2,3,4-tetrahydropyrimidine-5-carboxylate [(R)-2o]:

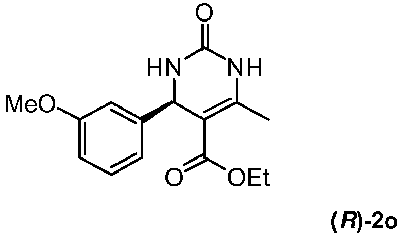

White solid, 81% yield; Mp 210°C (Lit. 219-220°C); ¹H NMR (300 MHz, DMSO-D6) δ: 1.10 (3H, t, *J* = 7.1, CH₂C*H*₃), 2.23 (3H, s, =CCH₃), 3.71 (3H, s, OCH₃), 3.99 (2H, q, *J* = 7.1, CH₂), 5.11 (1 H, d, *J* = 2.7, CHNH), 6.76-6.83 (3H, m, 3xArH), 7.20-7.27 (1H, m, ArH), 7.71 (1H, s, CHN*H*), 9.17 (1H, s, NHC=); ¹³C NMR (75.5 MHz, DMSO-D6) δ: 14.1 (CH₃CH₂), 17.7 (=C*C*H₃), 53.7, 54.9 (CHNH, CH₃O), 59.2 (CH₂), 99.1 (CO*C*=), 112.1, 112.4, 118.2, 129.5 (ArCH), 146.3, 148.4 (ArC, =CCH₃), 152.2 (NHCONH), 165.3 (COO).

Organocatalyst of formula *(R,R)-VI* used. The enantiomeric excess was determined by HPLC analysis (Chiralpak OD-H, hexane/*i*-PrOH = 8:2, 0.5 ml/min, 280 nm, *tᵣ* (minor) = 22.43 min, *tᵣ* (major) = 26.70 min 94% ee.

### Ethyl (R)-4-(furan-2-yl)-6-methyl-2-oxo-1,2,3,4-tetrahydropyrimidine-5-carboxylate [(R)-2p]:

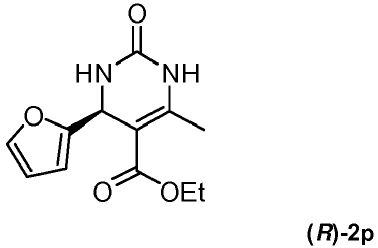

Light Brown solid, 99% yield; Mp 198°C (Lit. 209-211°C); ¹H NMR (300 MHz, DMSO-D6) δ: 1.13 (3H, t, *J =* 7.2, CH₂C*H*₃), 2.23 (3H, s, =CCH₃), 4.05 (2H, q, *J =* 7.2, CH₂C*H*₃), 5.20 (1 H, d, *J =* 3.3, CHNH), 6.09 (1 H, d, *J =* 3.3, CHFur), 6.34-6.37 (1 H, m, CHFur), 7.55-7.56 (1 H, m, CHFur), 7.76 (1 H, s, CHN*H*), 9.25 (1 H, s, NHC=); ¹³C NMR (75.5 MHz, DMSO-D6) δ: 14.2 (CH₃CH₂), 17.7 (=C*C*H₃), 47.7 (CHNH), 59.2 (CH₂), 96.8 (CO*C*=), 105.3, 110.4, 142.2 (FurCH), 149.4, 152.4 (=*C*CH₃, NHCONH), 155.9 (FurC), 165.0 (COO).

Organocatalyst of formula (*R,R*)-VII used. The enantiomeric excess was determined by HPLC analysis (Chiralpak OD-H, hexane/*i*-PrOH = 8:2, 0.5 ml/min, 254 nm, *tᵣ* (minor) = 18.57 min, *tᵣ* (major) = 21.10 min, 92% ee.

### Ethyl (R)-6-methyl-2-oxo-4-(thiophen-2-yl)-1,2,3,4-tetrahydropyrimidine-5-carboxylate [(R)-2q]:

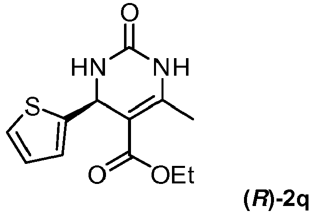

White solid, 79% yield; Mp 208°C (Lit. 209-210°C) ¹H NMR (300 MHz, DMSO-D6) δ: 1.16 (3H, t, *J =* 7.1, CH₂C*H*₃), 2.22 (3H, s, =CCH₃), 4.06 (2H, q, *J =* 7.1, CH₂), 5.41 (1 H, d, *J =* 3.3, CHNH), 6.87-6.95 (2H, m, 2×CHThio), 7.32-7.36 (1H, m, CHThio), 7.90 (1H, s, CHN*H*), 9.31 (1H, s, NHC=); ¹³C NMR (75.5 MHz, DMSO-D6) δ: 14.2 (CH₃CH₂), 17.7 (=C*C*H₃), 49.4 (CHNH), 59.4 (CH₂), 99.8 (CO*C*=), 123.5, 124.6, 126.7 (ThioCH), 148.7, 148.8 (ThioC, CCH₃), 152.2 (NHCONH), 165.0 (COO).

Organocatalyst of formula (*R,R*)-VII used. The enantiomeric excess was determined by HPLC analysis (Chiralpak OD-H, hexane/*i*-PrOH = 8:2, 0.5 ml/min, 280 nm, *tᵣ* (minor) = 19.06 min, *tᵣ* (major) = 22.16 min, 88% ee.

### Methyl (R)-6-methyl-2-oxo-4-phenyl-1,2,3,4-tetrahydropyrimidine-5-carboxylate [(R)-2r]:

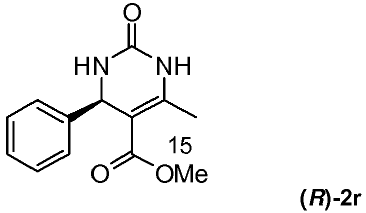

White solid, 99% yield; Mp 213°C (Lit. 209-212°C); ¹H NMR (300 MHz, DMSO-D6) δ: 2.26 (3H, s, =CCH₃), 3.53 (3H, s, OCH₃), 5.17 (1H, s, CH), 7.17-7.42 (3H, m, 3xArH), 7.78 (1H, s, CHN*H*), 9.25 (1H, s, NHC=); ¹³C NMR (75.5 MHz, DMSO-D6) δ: 17.9 (=C*C*H₃), 50.8 (OCH₃), 53.9 (CHNH), 99.1 (CO*C*=), 126.2, 127.4, 128.5 (ArCH), 144.7, 148.7 (=*C*CH₃, ArC), 152.3 (NHCONH), 165.9 (COO).

Organocatalyst of formula (*R*,*R*)-VI used. The enantiomeric excess was determined by HPLC analysis (Chiralpak OD-H, hexane/*i*-PrOH = 8:2, 0.5 ml/min, 254 nm, *tᵣ* (minor) = 17.98 min, *tᵣ* (major) = 22.25 min, 94% ee.

### Ethyl (R)-4-(3,4-difluorophenyl)-6-methyl-2-oxo-1,2,3,4-tetrahydropyrimidine-5-carboxylate [(R)-2s]:

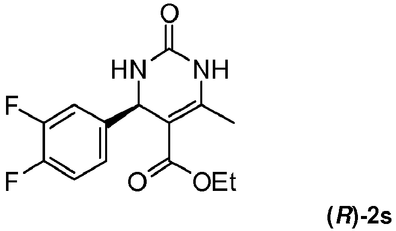

White solid, 88% yield; Mp 180°C (Lit. 185-186°C); ¹H NMR (300MHz, DMSO-D6) δ: 1.11 (3H, t, *J* = 7.1, CH₂C*H*₃), 2.30 (3H, s, =CCH₃), 3.95-4.11 (2H, m, CH₂), 5.20 (1 H, d, *J* = 3.3, CH), 7.10-7.14 (1H, m, ArH), 7.20-7.29 (1H, m, ArH), 7.35-7.45 (1H, m, ArH), 7.86 (1H, s, CHN*H*), 9.34 (1H, s, NHC=); ¹³C NMR (75.5 MHz, DMSO-D6) δ: 14.1 (CH₃CH₂), 17.9 (=C*C*H₃), 53.0 (CHNH), 59.4 (CH₂), 98.6 (CO*C*=), 115.3, 115.5, 117.4, 117.7, 122.9, 123.0, 123.0, 123.1 (ArCH), 142.6, 142.6, 142.7, 149.2, 152.0 (=*C*CH₃, ArC, NHCONH), 165.2 (COO).

Organocatalyst of formula (*R*,*R*)-VI used. The enantiomeric excess was determined by HPLC analysis (Chiralpak OD-H, hexane/*i*-PrOH = 8:2, 0.5 ml/min, 280 nm, *tᵣ* (minor) = 13.39 min, *tᵣ* (major) = 15.52 min, 96% ee.

### Methyl (R)-4-(3,4-difluoropheny)-6-methyl-2-oxo-1,2,3,4-tetrahydropyrimidine-5-carboxylate [(R)-2t]:

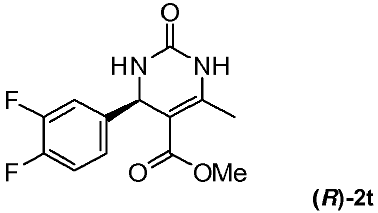

White solid, 83% yield; Mp 225°C (Lit. 224-226°C); ¹H NMR (300 MHz, DMSO-D6) δ: 2.31 (3H, s, =CCH₃), 3.57 (2H, s, CH₂), 5.22 (1 H, d, *J* = 2.4, CH), 7.13 (1 H, br s, ArH), 7.22-7.30 (1 H, m, ArH), 7.34-7.44 (1 H, m, ArH), 7.88 (1 H, s, CHN*H*), 9.38 (1 H, s, NHC=); ¹³C NMR (75.5 MHz, DMSO-D6) δ: 18.0 (=C*C*H₃), 50.9 (OCH₃), 53.2 (CHNH), 98.4 (CO*C*=), 115.3, 115.5, 117.5, 117.7, 122.9, 123.0 (ArCH), 142.4, 149.5, 152.1 (=*C*CH₃, ArC, NHCONH), 165.8 (COO).

Organocatalyst of formula (*R*,*R*)-VI used. The enantiomeric excess was determined by HPLC analysis (Chiralpak OD-H, hexane/*i*-PrOH = 8:2, 0.5 ml/min, 280 nm, *tᵣ* (minor) = 16.40 min, *tᵣ* (major) = 20.47 min, 92% ee.

### Methyl (S)-4-(3,4-difluorophenyl)-6-methyl-2-oxo-1,2,3,4-tetrahydropyrimidine-5-carboxylate [(S)-2t]:

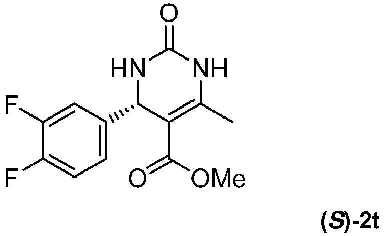

White solid, 98% yield; Mp 225°C (Lit. 224-226°C); ¹H NMR (300 MHz, DMSO-D6) δ: 2.31 (3H, s, =CCH₃), 3.57 (2H, s, CH₂), 5.22 (1H, d, *J* = 2.4, CH), 7.13 (1 H, br s, ArH), 7.22-7.30 (1 H, m, ArH), 7.34-7.44 (1 H, m, ArH), 7.88 (1 H, s, CHN*H*), 9.38 (1 H, s, NHC=); ¹³C NMR (75.5 MHz, DMSO-D6) δ: 18.0 (=C*C*H₃), 50.9 (OCH₃), 53.2 (CHNH), 98.4 (CO*C*=), 115.3, 115.5, 117.5, 117.7, 122.9, 123.0 (ArCH), 142.4, 149.5, 152.1 (=*C*CH₃, ArC, NHCONH), 165.8 (COO).

Organocatalyst of formula (*S,S*)-VI used. The enantiomeric excess was determined by HPLC analysis (Chiralpak OD-H, hexane/*i*-PrOH = 8:2, 0.5 ml/min, 280 nm, *tᵣ* (major) = 16.62 min, *tᵣ* (minor) = 21.61 min, 84% ee.

### Ethyl (R)-6-methyl-2-oxo-4-(3-(trifluoromethyl)phenyl)-1,2,3,4-tetrahydropyrimidine-5-carboxylate [(R)-2u]:

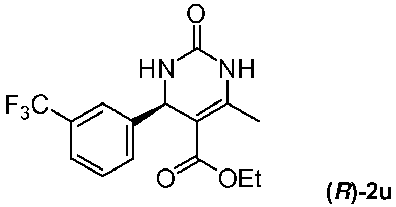

White solid, 95% yield; Mp 171°C; ¹H NMR (300 MHz, DMSO-D6) δ: 1.09 (3H, t, *J* = 6.9, CH₂C*H*₃), 2.29 (3H, s, =CCH₃), 3.93-4.13 (2H, m, CH₂), 5.29 (1 H, s, CH), 7.56-7.66 (4H, m, 4xArH), 7.88 (1H, s, CHN*H*), 9.36 (1H, s, NHC=); ¹³C NMR (75.5 MHz, DMSO-D6) δ: 13.9 (CH₃CH₂),17.8 (=C*C*H₃), 53.9 (CHNH), 59.3 (CH₂), 98.6 (CO*C*=), 122.4, 123.0 (ArC), 123.1, 124.1 (ArCH), 124.1, 126.1, 128.8, 129.3, 129.6 (ArC), 129.8, 130.4 (ArCH), 146.3, 149.2, 152.0 (=*C*CH₃, ArC, NHCONH), 165.2 (COO).

Organocatalyst of formula (*R*,*R*)-VI used. The enantiomeric excess was determined by HPLC analysis (Chiralpak OD-H, hexane/*i*-PrOH = 8:2, 0.5 ml/min, 280 nm, *tᵣ* (minor) = 13.93 min, *tᵣ* (major) = 16.97 min, 83% ee.

### EXAMPLE 3: Medium scale, "one pot" enantioselective preparation process of DHPMs from α-ureidosulfones

To a suspension of the α-ureidosulfone (2 mmoles) **1a, 1o, 1f, 1e** or **1t** of Example 1 in dichloromethane (20 ml), cooled down to 0°C, N,N'-diisopropylethylamine (8 mmoles, 1.41 ml, 4 equivalents) is added dropwise, followed by a dichloromethane solution (20 ml) of the organocatalyst of formula VI: 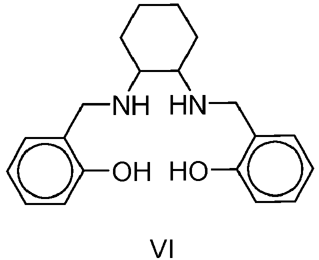 diastereomerically and enantiomerically enriched with one of the (*R,R*) or (*S,S*) stereoisomers, (0.2 mmoles, 0.1 equivalents) and ethyl or methyl acetoacetate (2.1 mmoles). Stirring at 0°C is maintained for 20 hours. After this, 5M HCl (20ml) is added to the two-phase mixture and stirred at room temperature for another 5 hours. Brine (170 ml) is added, and the mixture is extracted three times with ethyl acetate (3x150ml). The combined organic phases are dried with anhydrous MgSO₄, then filtered and the solvent rotoevaporated under vacuum. The crude solid material is then washed with diethyl ether and hexane and checked in HPLC for enantiomeric purity (typically using a Chiralpak OD-H chiral column from Daicel) prior to recrystallization.

The enantiomerically enriched DHPMs **2a, 2o, 2f, 2e** (using ethyl acetoacetate) and **2t** (using methyl acetoacetate). were synthesized according to this example. The (*R*) or (*S*) configuration of the DHPM synthesized in enantiomeric excess is featured in each case. Enantiomerically enriched DHPMs **(*R*)-2a, (*R*)-2o** and **(*R*)-2f** were synthetized making use of the (*R,R*) enantiomer of the organocatalyst VI, and enantiomerically enriched DHPMs **(*S*)-2e** and **(*S*)-2t** were synthetized making use of the (*S,S*) enantiomer of the organocatalyst VI:

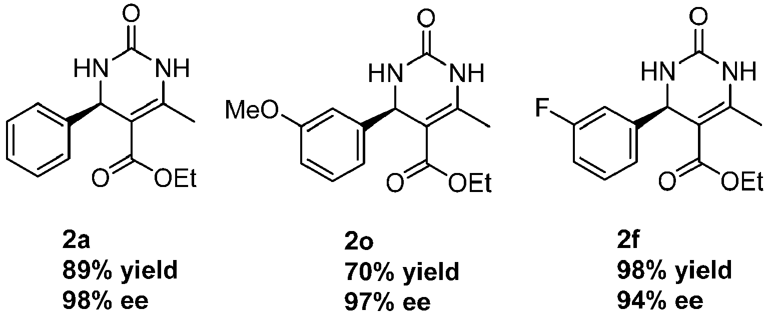

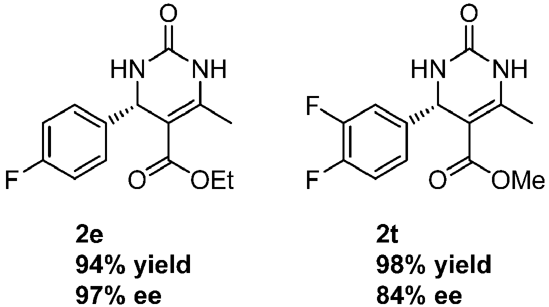

### EXAMPLE 4: Organocatalyst recovery and reuse

An acid-base treatment allowed the recovery of the organocatalyst of formula VI (*R*,*R*) used in the medium scale preparation process in a high yield (ca. 90%) and purity. This recovered organocatalyst was reused in a further medium scale synthesis of DHPM **(*R*)-2a** which led to almost identical results (89% yield and 98% ee in the first synthesis and 96% yield and 93 % ee in the second).

### EXAMPLE 5: Synthesis of enantiomerically and diastereomerically enriched HHPMs from α-ureidosulfones

In a round-bottom flask with a magnetic stirrer, N,N'-diisopropylethylamine (0.8 mmoles, 4 eq.) was added dropwise to a cooled (0°C) suspension of the α-ureido sulfone II, **1a** or **1e,** (0.2 mmoles) in dicloromethane (2 ml). A solution of the organocatalyst (*R*,*R*)-VI (0.02 mmoles, 7 mg, 0.1 equivalents) and ethyl acetoacetate (0.21 mmoles) in 2 ml of dicloromethane was then slowly added to the above mixture. The resulting suspension was then stirred at 0°C for 15-20 hours after which time the mixture becomes a fully transparent solution. Then tetramethylguanidine (0.8 mmoles, 4 eq.) was added and the mixture stirred at room temperature for an additional 20 hours. To the resulting mixture 15 ml of a saturated solution of NaCl in water was added, followed by extraction with AcOEt (3×15 ml). The combined organic extracts were dried over anhydrous MgSO₄, filtered and evaporated under vacuum. Diethyl ether was added to the resulting crude and then hexane, thereby giving rise to the formation of an abundant precipitate of pure HHPMs.

### Ethyl (4R,5R,6S)-4-hydroxy-4-methyl-2-oxo-6-phenylhexahydropyrimidine-5-carboxylate (3a):

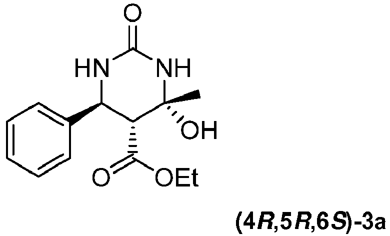

White solid, 84% yield (dr = 88:12, 76% de); Mp 154°C; ¹H NMR (300 MHz, DMSO D6) δ: 0.93 (3H, t, *J* = 7.2, CH₂C*H*₃), 1.40 (3H, s, CCH₃), 1.40 (3H, s, CCH₃ minor diastereomer (*4S,5R,6S*))*,* 2.73 (1 H, d, *J* = 11.4, COCH), 2.91 (1 H, d, *J* = 11.4, COCH minor diastereomer (*4S,5R,6S*))*,* 3.78-3.98 (2H, m, CH₂), 4.50 (1H, d, *J* = 11.4, PhCH minor diastereomer (*4S,5R,6S*))*,* 4.80 (1 H, d, *J =* 11.4, PhCH), 5.66 (1 H, s, OH), 6.64 (1 H, s, NH), 7.13 (1 H, s, NH), 7.20-7.40 (5H, m, 5xArH); ¹³C NMR (75.5 MHz, CDCl₃) δ: 13.9, 14.0 (CH₃CH₂), 28.0 (CHCH₃), 54.9, 56.0 (CHCO, CHNH), 61.3 (CH₂), 79.4 (COH), 127.7, 128.8, 128.9 (ArCH), 138.6 (ArC), 155.9 (NHCONH), 170.7 (COO).

The enantiomeric excess was determined by HPLC analysis (Chiralpak IC, hexane/EtOH = 7:3, 0.5 ml/min, 210 nm, *tᵣ* (minor) = 20.00 min, *tᵣ* (major) = 60.55 min, 93% ee. The diasteromeric excess (de) was 76%

### Ethyl (4R,5R,6S)-6-(4-fluorophenyl)-4-hydroxy-4-methyl-2-oxohexahydropyrimidine-5-carboxylate (3e):

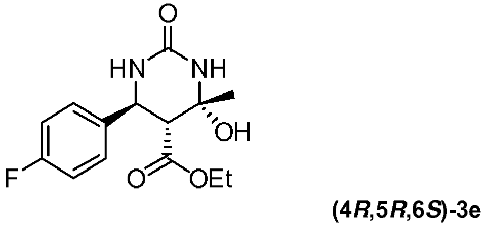

White solid, 88% yield (dr = 85:15, 70% de); Mp 191°C; ¹H NMR (300 MHz, CDCl₃) δ: 0.97 (3H, t, *J =* 7.2, CH₂C*H*₃), 1.52 (3H, s, CCH₃), 2.80 (1 H, d, *J =* 11.4, COCH), 3.05 (1 H, d, *J* = 11.4, COCH minor diastereomer(*4S*,*5R*,*6S*)) 3.89-4.02 (2H, m, CH₂), 4.93 (1H, d, *J =* 11.4, PhCH), 5.34 (1 H, s, NH), 6.46 (1 H, s, NH), 6.98-7.06 (2H, m, 2xArH), 7.25-7.36 (2H, m, 2xArH); ¹³C NMR (75.5 MHz, DMSO-D6) δ: 13.9, 13.9 (CH₃CH₂), 27.6 (CHCH₃), 52.7, 56.4 (CHCO, CHNH), 59.9 (CH₂), 78.6 (COH), 115.0, 114.7, 129.9, 130.1, 130.4 (ArCH), 136.8, 136.9 (ArC), 154.5, 160.0, 163.3 (=*C*CH₃, ArC, NHCONH), 168.8 (COO).

The enantiomeric excess was determined by HPLC analysis (Chiralpak IC, hexane/EtOH = 7:3, 0210 nm, *tᵣ* (minor) = 17.62 min, *tᵣ* (major) = 43.21 min, 90% ee. The diasteromeric excess (de) was 70%

### EXAMPLE 6: Medium scale, "one pot" enantioselective preparation process of HHPDs from α-ureidosulfones

To a suspension of the α-ureidosulfone (2 mmoles) 1a of Example 1 in dichloromethane (20 ml), cooled down to 0°C, N,N'-diisopropylethylamine (8 mmoles, 1.41 ml, 4 equivalents) is added dropwise, followed by a dichloromethane solution (20 ml) of the organocatalyst (*R*,*R*)-VI (0.2 mmoles, 0.1 equivalents) and ethyl acetoacetate (2.1 mmoles). Stirring at 0°C is maintained for 20 hours. After this, tetramethylguanidine (4 mmoles, 0.51 ml, 2 equivalents) is added and the mixture stirred at room temperature for another 20 hours. Brine (170 ml) is added, and the mixture is extracted three times with ethyl acetate (3x100ml). The combined organic phases are dried with anhydrous MgSO₄, then filtered and the solvent rotoevaporated under vacuum. Diethyl ether was added to the resulting crude and then hexane, thereby giving rise to the formation of a precipitate of pure HHPMs.

The enantiomerically enriched HHPMs **(4*R*,5*R*,6*S*)-3a** was synthesized according to this example making use of the (*R*, *R*) enantiomer of the organocatalyst VI:

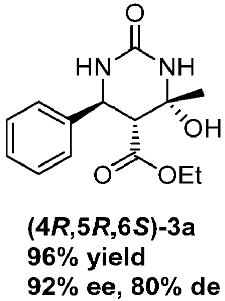

## Claims

1. A preparation process of a reaction crude comprising a compound of formula I: 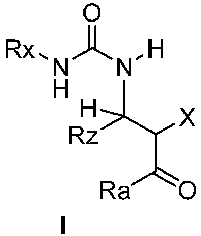 **characterized by** adding a compound of formula X-CH₂-C(O)Rₐ to a compound of formula II 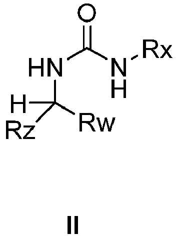 in the presence of a diastereomerically and enantiomerically enriched organocatalyst comprising a network of cooperative hydrogen bonds (NCHB) of formula III, a base and a solvent, wherein said preparation process is conducted maintaining a reaction temperature of between -80°C and 85°C;
(i) wherein
- Rₓ is selected independently from H, alkyl, aryl, heteroaryl or a fused aryl comprising 0-5 heteroatoms selected independently from nitrogen, oxygen or sulfur;
- R_{z} is selected independently from H, heteroaryl, phenyl, substituted aryl, fused aryl comprising 0-5 heteroatoms selected independently from nitrogen, oxygen or sulfur; or a group -C(R")₃, wherein each group R" is independently selected from halogen, alkyl or aryl;
- R_{w} is independently selected from halogen,-CN, -OCN, -SCN, -ONC, -SNC, -OR', -OC(O)R', -OC(O)OR', -OC(O)SR', -OC(S)OR', -OC(S)SR', -OC(O)N(R')₂, -OC(NR')N(R')₂, -OP(R')₂, -OP(O)R', -OP(O)OR', -OP(O)(OR')₂, -OP(S)R', -OP(S)OR', -OP(S)(OR')₂, -OP(S)SR', -OP(S)(SR')₂, -OP(O)N(R')₂, -OP(O)(NR')₂, -OP(S)N(R')₂, -OP(S)(NR')₂, -OP(O)₂OR', -OP(O)₂SR', -OP(O)₂N(R')₂, -OP(O)₂OP(O)₂OR', -OP(O)₂OP(O)₂SR', -OP(O)₂OP(O)₂N(R')₂, -OP(O)₂OP(O)₂OP(O)₂OR', -OP(O)₂OP(O)₂OP(O)₂SR', -OP(O)₂OP(O)₂OP(O)₂N(R')₂, -SR', -S(O)R', -S(O)₂R', -S(O)(NR')R', -S(O)₂OR', -S(O)₂N(R')₂, -S(NR')(NR')R', -S(S)₂R', -S(S)(NR')R', -S(S)₂R', -S(S)₂OR', -S(S)₂N(R')₂, -NHR', -N(R')₂, -NHC(O)R', -NR'C(O)R', -NR'C(O)OR', -NR'C(O)N(R')₂, -NR'C(S)R', -NR'C(S)OR', -NR'C(S)N(R')₂, -NHC(O)OR', -NHC(O)N(R')₂, -NHC(S)R', -NHC(S)OR', -NHC(S)N(R')₂, -N₃, -P(R')₂, -P(O)(R')₂, -P(OR')₂, -P(O)(OR')₂, -P(O)[N(R')₂]₂, -P(O)(OR')[N(R')₂], -P(S)(R')₂, -P(S)(OR')₂, -P(S)(SR')₂, or -P(S)[N(R')₂]₂; wherein each R' is independently selected from hydrogen, a metal, alkyl, halogen-alkyl, alkenyl, phenyl or substituted aryl;
- X is selected independently from -C(O)OR, -C(O)SR, -C(S)OR, -C(S)SR, -C(O)NH₂, -C(O)NHR, -C(O)N(R)₂, -C(O)NHOR, -C(O)NROR, -C(S)NH₂, -C(S)NHR, -C(S)N(R)₂, -C(S)NHOR, -C(S)NROR, -P(O)(OR)₂, -P(O)OR, -P(O)(SR)₂, -P(O)SR, -P(S)(SR)₂, -P(S)SR, -P(O)(NH₂)₂, -P(O)(NHR)₂, -P(O)[N(R)₂]₂, -P(S)(NH₂)₂, -P(S)(NHR)₂, -CN, -NO₂, -SO₂R, -SOR or -P(S)[N(R)₂]₂, wherein each R is independently selected from alkyl, halogen-alkyl, alkenyl, a saturated or partially unsaturated cycle, a saturated or partially unsaturated heterocycle, an aryl, an heteroaryl or a fused aryl comprising 0-5 heteroatoms selected independently from nitrogen, oxygen or sulfur; and
- Rₐ is selected independently from H, alkyl, halogen-alkyl, alkenyl, alkylalkoxy, alkylamine, alkylammonium, alkylphosphine, alkylphosphonium, a saturated or partially unsaturated cycle, a saturated or partially unsaturated heterocycle, an aryl, an heteroaryl or a fused aryl comprising 0-5 heteroatoms selected independently from nitrogen, oxygen or sulfur; and
(ii) wherein in said diastereomerically and enantiomerically enriched chiral organocatalyst comprising a NCHB of formula III 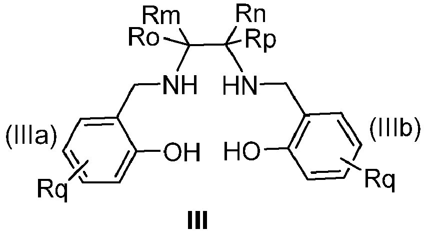
- Rₘ, and Rₙ, or Rₒ and Rₚ, are linked forming a saturated or partially unsaturated C₃₋₁₂ ring; or are each independently selected from H, alkyl, aryl or heteroaryl; and
- Rq is selected independently from H, hydroxyl, thiol, sulfone, amine, nitro, halogen, cyano, alkyl, alkylalkoxy, aryl or heteroaryl; or wherein each radical Rq of rings (IIIa) and (IIIb) is an aryl or an heteroaryl ring fused to each of said aryl rings (IIIa) and (IIIb) by two contiguous positions.

2. The preparation process according to claim 1 wherein the diastereomerically and enantiomerically enriched organocatalyst comprising a NCHB of formula III is selected from:

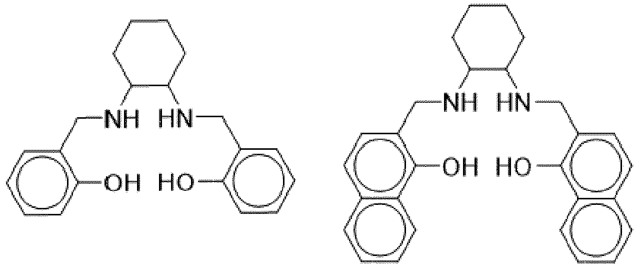

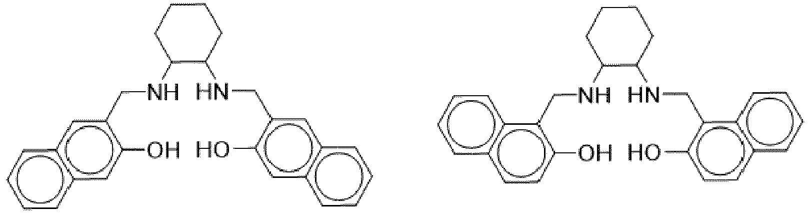

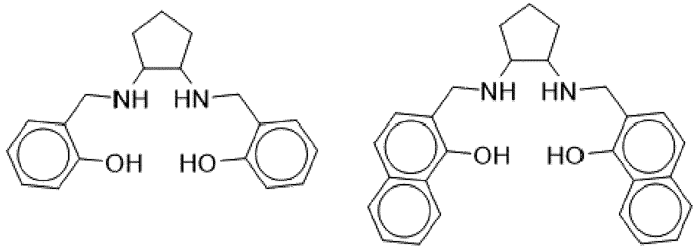

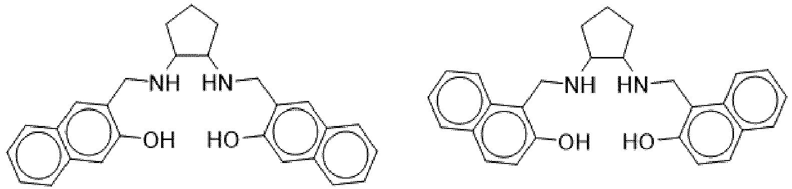

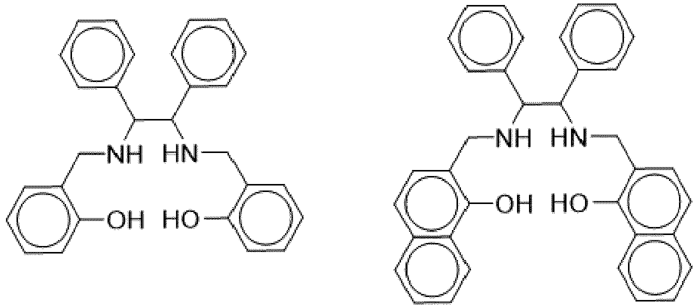

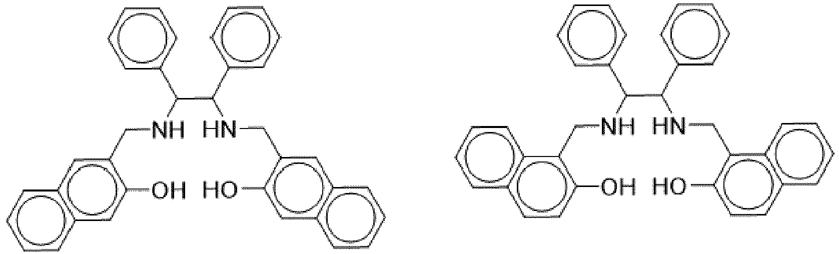

3. The preparation process according to any of claims 1 or 2, wherein the amount of the diastereomerically and enantiomerically enriched organocatalyst comprising a NCHB of formula III is lower than 1 mole equivalent relative to compound of formula II.

4. The preparation process according to any of claims 1-3, wherein Rₓ is H.

5. The preparation process according to any of claims 1-4, wherein
- when R_{z} is a substituted aryl, said aryl is substituted with one or more groups R_{b} 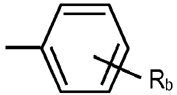 wherein R_{b} is independently selected from alkyl, alkoxy, halogen-alkyl, halogen, cyano, primary amine, secondary amines, tertiary amines or nitro; and;
- when R_{z} is a fused aryl comprising 0-5 heteroatoms selected independently from nitrogen, oxygen or sulfur, said fused aryl is naphtyl.

6. The preparation process of any of claims 1-5, wherein R_{w} is -S(O)₂R' and R' is tolyl.

7. The preparation process according to any of claims 1-6, wherein the base is N,N'-diisopropylethylamine.

8. The preparation process according to any of claims 1-7, wherein X is -C(O)OR and wherein R is selected from a methyl or an ethyl group

9. The preparation process according to any of claims 1-8, wherein Rₐ is methyl or ethyl.

10. An enantioselective preparation process of enantiomerically enriched dihydropyrimidinones (DHPMs) of formula IV: 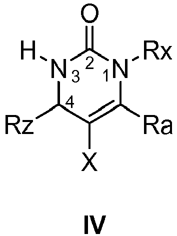 wherein said process comprises
a) obtaining the reaction crude of the preparation process of compound of formula I according to any of claims 1-9;
b) adding an acid to the reaction crude of step (a); and
wherein one DHPM enantiomer is obtained with enantiomeric excess greater than 0%.

11. The preparation process according to claim 10 wherein the reaction is conducted maintaining a temperature of between -10°C and 10°C during at least 8 hours.

12. The preparation process according to any of claims 10 or 11 wherein step (b) is conducted adding a hydrochloric acid solution to the reaction crude of step (a).

13. Enantiomerically and diastereomerically enriched Hexahydropyrimidinones (HHPMs) of formula V 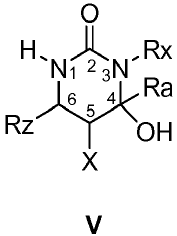 wherein
- Rₓ is selected independently from H, alkyl, aryl, heteroaryl or a fused aryl comprising 0-5 heteroatoms selected independently from nitrogen, oxygen or sulfur;
- R_{z} is selected independently from H, heteroaryl, phenyl, substituted aryl, fused aryl comprising 0-5 heteroatoms selected independently from nitrogen, oxygen or sulfur; or a group -C(R")₃, wherein each group R" is independently selected from halogen, alkyl or aryl;
- X is selected independently from -C(O)OR, -C(O)SR, -C(S)OR, -C(S)SR, -C(O)NH₂, -C(O)NHR, -C(O)N(R)₂, -C(O)NHOR, -C(O)NROR, -C(S)NH₂, -C(S)NHR, -C(S)N(R)₂, -C(S)NHOR, -C(S)NROR, -P(O)(OR)₂, -P(O)OR, -P(O)(SR)₂, -P(O)SR, -P(S)(SR)₂, -P(S)SR, -P(O)(NH₂)₂, -P(O)(NHR)₂, -P(O)[N(R)₂]₂, -P(S)(NH₂)₂, -P(S)(NHR)₂, -CN, -NO₂, -SO₂R, -SOR or -P(S)[N(R)₂]₂, wherein each R is independently selected from alkyl, halogen-alkyl, alkenyl, a saturated or partially unsaturated cycle, a saturated or partially unsaturated heterocycle, an aryl, an heteroaryl or a fused aryl comprising 0-5 heteroatoms selected independently from nitrogen, oxygen or sulfur; and
- Rₐ is selected independently from H, alkyl, halogen-alkyl, alkenyl, alkylalkoxy, alkylamine, alkylammonium, alkylphosphine, alkylphosphonium, a saturated or partially unsaturated cycle, a saturated or partially unsaturated heterocycle, an aryl, an heteroaryl or a fused aryl comprising 0-5 heteroatoms selected independently from nitrogen, oxygen or sulfur.

14. The enantiomerically and diastereomerically enriched HHPMs of formula V of claim 13, wherein
- Rₓ is H;
- X is -C(O)OR and wherein R is ethyl;
- Rₐ is methyl; and
- R_{z} is phenyl or 4-fluorophenyl.

15. An preparation process of the enantiomerically and diastereomerically enriched HHPMs of formula V of any of claims 13 or 14, wherein said process is **characterized by**:
a) obtaining the reaction crude comprising the compound of formula I according to any of claims 1-9;
b) adding a base to the reaction crude of step (a); and
wherein one HHPM stereoisomer is obtained with a diastereomeric excess greater than 0% and an enantiomeric excess greater than 0%.
